# EUROPEAN PATENT APPLICATION

(11) **EP 4 759 798 A1**
(43) Date of publication of application: **17.06.2026**
(21) Application number: 24864726.5
(22) Date of filing: 13.09.2024
(51) Int. Cl.: C07C 311/29, C07D 231/12, C07D 231/42, C07D 241/22, A61K 31/18, A61P 11/00, A61P 29/00

(54) **NEUTROPHIL ELASTASE INHIBITOR AND USE THEREOF**

(30) Priority: 15.09.2023 CN 202311189527; 12.12.2023 CN 202311696293; 18.02.2024 CN 202410180987
(71) Applicant: Shanghai Yidi Biotechnology Co., Ltd., Shanghai 201208 (CN)
(72) Inventor: YE, Bin, Shanghai 201208 (CN); LI, Wenhua, Shanghai 201208 (CN); TIAN, Songchuan, Shanghai 201208 (CN); YU, Jun, Shanghai 201208 (CN)
(74) Representative: Regimbeau
(86) International application number: PCT/CN2024/118671
(87) International publication number: WO 2025/056012

(57) **Abstract**

The present invention relates to a neutrophil elastase inhibitor and the use thereof, a preparation method therefor, a pharmaceutical composition, and the use thereof in the treatment of related diseases caused by an abnormal increase of neutrophil elastase. The compound of the present invention has a good inhibitory activity against neutrophil elastase.

## Description

### TECHNICAL FIELD

The present invention relates to the field of medicines and specifically a neutrophil elastase inhibitor and use thereof, a preparation method, a pharmaceutical composition, and use thereof in the treatment of diseases caused by abnormal increase of neutrophil elastase.

### BACKGROUND

Neutrophil elastase (NE), a serine protease, is mainly distributed in the azurophilic granules of neutrophils. In an acute phase of inflammation, neutrophils are the first cells recruited to the inflammatory site and form the earliest defense against invasion. NE can degrade a variety of extracellular matrix proteins, such as elastin, proteoglycan, laminin and fibronectin. In a normal physiological process, NE can degrade or repair damaged tissues by degrading these tissue structural proteins, to maintain tissue homeostasis. NE can also promote the recruit of neutrophils to the inflammatory site by degrading structural proteins of bacteria, thus reducing the bacterial infection and participating in the regulation of inflammation.

However, the excessive release and enrichment of NE can destroy the normal physiological tissue structure, and also induce increased microvascular permeability and excessive mucus secretion. In addition, the excessive release of NE may also lead to some serious cardiopulmonary diseases. Diseases related to NE overexpression include acute lung injury (ALI), acute respiratory distress syndrome (ARDS), and acute lung injury (ALI)/ARDS with systemic inflammatory response syndrome (SIRS).

In the studies over past years, researchers have designed and developed many small molecular NE inhibitor compounds with different types of structures. The small molecular NE inhibitor compound, including pivalate derivatives, such as Sivelestat (having an inhibitory activity against neutrophil elastase of 44 nM) disclosed in Document 1, is the first drug in the world to treat ALI/ARDS with SIRS. In addition, many studies and reports also prove that Sivelestat has therapeutic and protective effects in the treatment of sepsis, cystic fibrosis, chronic obstructive pulmonary disease (COPD) and ischemia-reperfusion injury of various tissues. Other types of small molecular NE inhibitors include pyridone derivatives (such as Alvelestat), dihydropyrimidinone derivatives (such as BAY 85-8501), small molecular polypeptides (such as POL6014) and so on.

It is well known in the art that subtle differences in the compound structures may lead to great differences in activities. Based on the prior art, the present invention develops a new compound with excellent NE inhibitory activity, which can be used in the treatment of diseases caused by abnormal increase of NE.

Document 1: EP 0347168 B1

### SUMMARY

In a first aspect, the present invention provides a compound of Formula I: 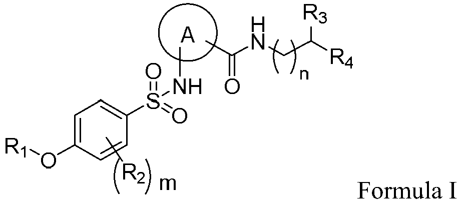
or a pharmaceutically acceptable salt thereof,
   where
R₁ is selected from (1) R₆-C(=O)-; or (2) R₁ is attached to a benzene ring to form a five-membered or six-membered ring, in which the five-membered or six-membered ring is optionally substituted with R₆;
R₆ is selected from t-butyl, C₃-C₆ cycloalkyl, and C₃-C₆ heterocycloalkyl;
ring A is selected from 5 to 10-membered cycloalkyl, heterocycloalkyl, heteroaryl, or aryl, and ring A is optionally substituted with one or more R₅;
R₂ and R₅ are each independently selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl;
R₃ is selected from hydrogen, C₁-C₆ alkyl, C₁-C₆ alkoxy, C₃-C₆ cycloalkyl, and C₁-C₆ haloalkyl;
R₄ is optionally selected from -COOH, -SO₃H, and -PO₃H₂; and
m and n are independently 0, 1, 2, and 3;
where the alkyl, alkoxy, cycloalkyl, heterocycloalkyl, and heteroaryl are unsubstituted or substituted with one or more substituents selected from: deuterium, C₁-C₃ alkyl, C₁-C₃ alkoxy, C₁-C₃ deuterated alkoxy, halogen, cyano, trifluoromethyl, nitro, amino, oxo, -CONH₂, and -COOH.

In some embodiments of the present invention, ring A is a 5 to 6-membered ring optionally containing 1 to 3 heteroatoms, where the heteroatom includes nitrogen atom, oxygen atom, and sulfur atom.

In some embodiments of the present invention, ring A is phenyl, pyrazolyl, imidazolyl, pyrazinyl, or cyclohexyl, and ring A is unsubstituted or optionally substituted with one or more R₅; and R₅ is optionally selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl.

In some embodiments of the present invention, R₅ is optionally selected from hydrogen, halogen, cyano, trifluoromethyl, nitro, amino, imidazolyl, pyrazolyl, oxazolyl, or isoxazolyl, wherein imidazolyl, pyrazolyl, oxazolyl, and isoxazolyl are unsubstituted or substituted with alkyl.

In some embodiments of the present invention, ring A is phenyl, pyrazolyl, imidazolyl, pyrazinyl, or cyclohexyl, and ring A is unsubstituted or optionally substituted with one or more R₅; and R₅ is optionally selected from hydrogen, halogen, cyano, trifluoromethyl, nitro, amino, methyl, methoxy, imidazolyl, pyrazolyl, or isoxazolyl, wherein imidazolyl, pyrazolyl, and isoxazolyl are unsubstituted or substituted with methyl.

In some embodiments of the present invention, R₆ is t-butyl.

In some embodiments of the present invention, R₆ is cyclopropyl, cyclobutyl, or azetidinyl, where the cyclopropyl, cyclobutyl, and azetidinyl are optionally substituted with one or more halogen or methyl.

In some embodiments of the present invention, R₆ is cyclopropyl, oxetanyl or azetidinyl, which are optionally substituted with one or more fluorine or methyl.

In another aspect, the present invention provides a compound of Formula I or a pharmaceutically acceptable salt thereof. Formula I has a structure of Formula I-a: 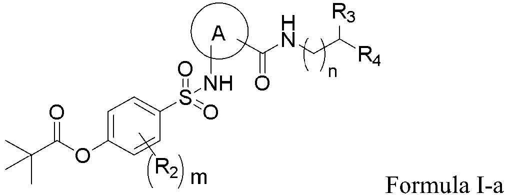 where
ring A is a 5 to 6-membered ring optionally containing 1 to 3 heteroatoms, and ring A is optionally substituted with one or more R₅;
R₄ is selected from -COOH, -SO₃H, and -PO₃H₂;
R₂ and R₅ are each independently selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl;
R₃ is selected from hydrogen, C₁-C₆ alkyl, C₁-C₆ alkoxy, C₃-C₆ cycloalkyl, and C₁-C₆ haloalkyl; and
m and n are independently 0, 1, 2, and 3,
where the alkyl, alkoxy, cycloalkyl, heterocycloalkyl, and heteroaryl are unsubstituted or optionally substituted with one or more substituents selected from: deuterium, C₁-C₃ alkyl, C₁-C₃ alkoxy, C₁-C₃ deuterated alkoxy, halogen, cyano, trifluoromethyl, nitro, amino, -CONH₂, and -COOH.

In some embodiments of the present invention, ring A in Formula I-a is phenyl, pyrazolyl, imidazolyl, pyrazinyl, or cyclohexyl, and ring A is optionally substituted with one or more R₅.

In another aspect, the present invention provides a compound of Formula I or a pharmaceutically acceptable salt thereof. Formula I has a structure of Formula I-b: 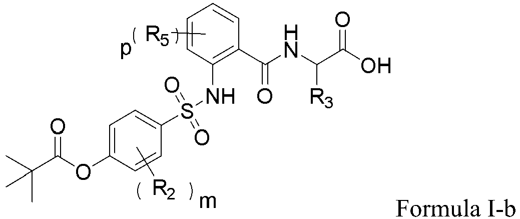
where R₂ and R₅ are each independently selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl;
R₃ is selected from hydrogen, C₁-C₆ alkyl, C₁-C₆ alkoxy, C₃-C₆ cycloalkyl, and C₁-C₆ haloalkyl; and
m and p are each independently 0, 1 or 2;
provided that R₂, R₃ and R₅ are not all hydrogen,
where the alkyl, alkoxy, cycloalkyl, heterocycloalkyl, and heteroaryl are unsubstituted or optionally substituted with one or more substituents selected from: deuterium, C₁-C₃ alkyl, C₁-C₃ alkoxy, C₁-C₃ deuterated alkoxy, halogen, cyano, trifluoromethyl, nitro, and amino.

In some embodiments of the present invention, R₂ in Formula I-b is optionally selected from hydrogen and fluorine.

In some embodiments of the present invention, R₃ in Formula I-b is optionally selected from hydrogen, methyl, fluoromethyl, methoxymethyl, and deuterated methoxymethyl.

In some embodiments of the present invention, R₅ in Formula I-b is optionally selected from hydrogen, fluorine, methyl, cyano, methoxy, imidazolyl, pyrazolyl, oxazolyl, or isoxazolyl where the imidazolyl, pyrazolyl, oxazolyl and isoxazolyl are unsubstituted or substituted with alkyl.

In another aspect, the present invention provides a compound of Formula I or a pharmaceutically acceptable salt thereof. Formula I has a structure of Formula I-c: 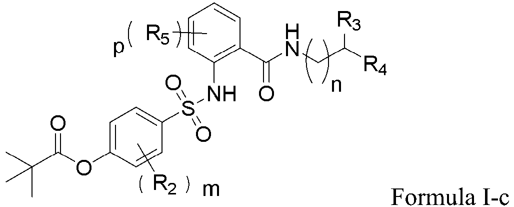
where in Formula I-c,
R₄ is optionally selected from -SO₃H and -PO₃H₂;
R₂ and R₅ are each independently selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl;
R₃ is selected from hydrogen, C₁-C₆ alkyl, C₁-C₆ alkoxy, C₃-C₆ cycloalkyl, and C₁-C₆ haloalkyl; and
n, m, and p are each independently 0, 1 or 2;
where the alkyl, alkoxy, cycloalkyl, heterocycloalkyl, and heteroaryl are unsubstituted or optionally substituted with one or more substituents selected from: deuterium, C₁-C₃ alkyl, C₁-C₃ alkoxy, C₁-C₃ deuterated alkoxy, halogen, cyano, trifluoromethyl, nitro, amino, -CONH₂, and -COOH.

In some embodiments of the present invention, in Formulas I, I-a, and I-c, R₄ is optionally selected from -SO₃H and -PO₃H₂.

In some embodiments of the present invention, in Formulas I and I-a, ring A is phenyl, pyrazolyl, imidazolyl, pyrazinyl, or cyclohexyl, and ring A is unsubstituted or optionally substituted with one or more R₅.

In some embodiments of the present invention, in Formulas I, I-a, I-b, and I-c, R₃ is hydrogen, methyl, fluoromethyl, methoxymethyl, and deuterated methoxymethyl.

In some embodiments of the present invention, in Formulas I, I-a, I-b, and I-c, R₂ is hydrogen, fluorine, cyano, trifluoromethyl, nitro, and amino.

In some embodiments of the present invention, in Formulas I, I-a, I-b, and I-c, R₅ is hydrogen, fluorine, cyano, trifluoromethyl, nitro, amino, imidazolyl, pyrazolyl, oxazolyl, or isoxazolyl, where the imidazolyl, pyrazolyl, oxazolyl, and isoxazolyl are unsubstituted or substituted with methyl.

In some embodiments of the present invention, in Formulas I, I-a, I-b, and I-c, where a chiral center is present, the configuration can be an R configuration, an S configuration or a racemate.

The compound of Formula I according to the present invention or the pharmaceutically acceptable salt thereof has the following structure:

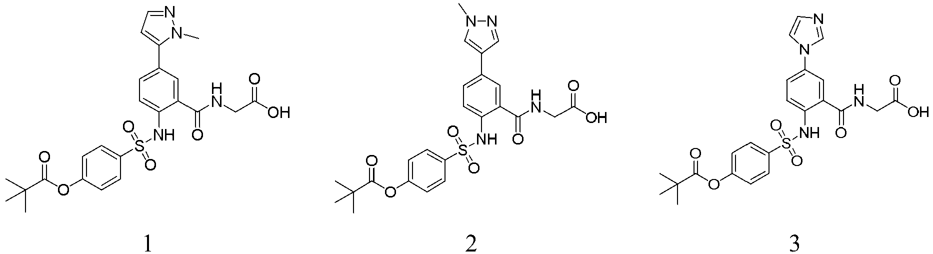

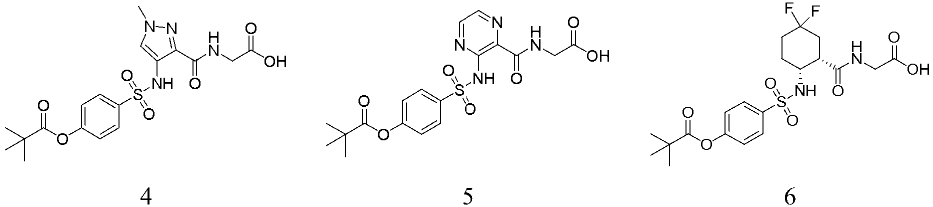

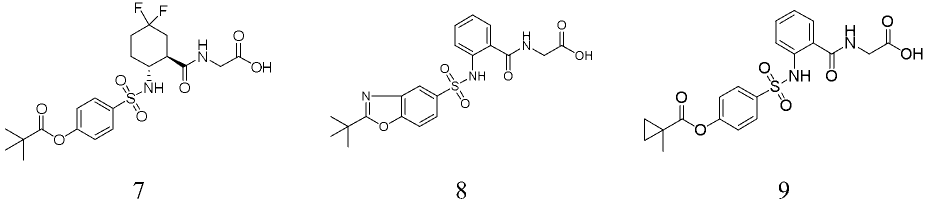

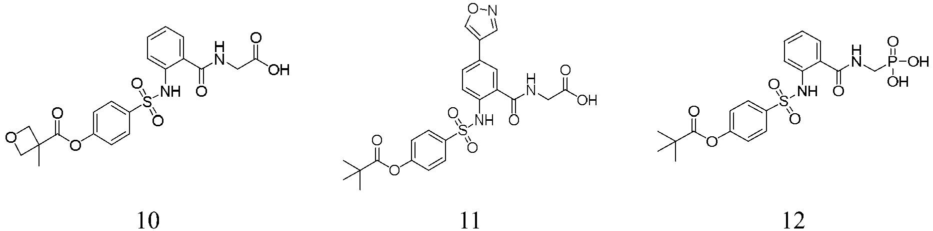

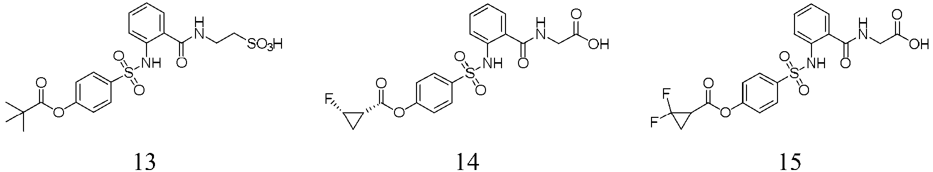

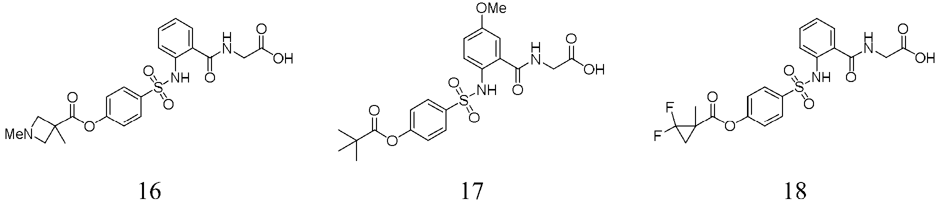

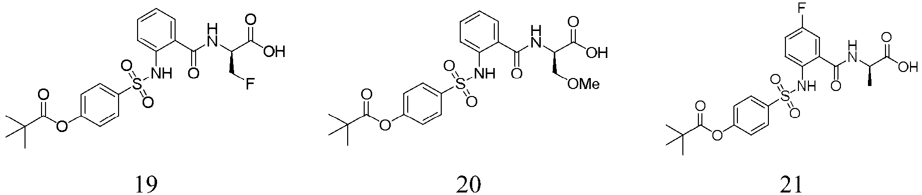

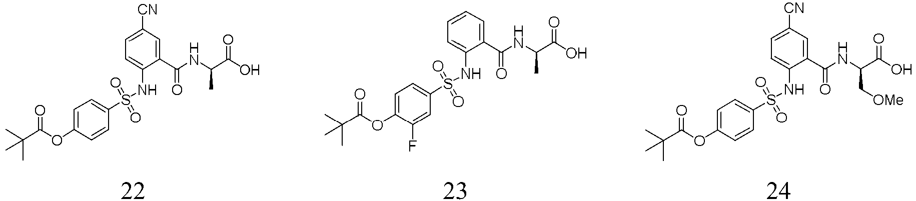

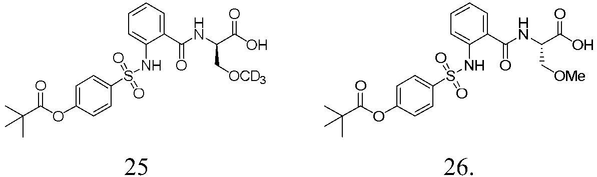

In another aspect of the present invention, a pharmaceutical composition is provided. The pharmaceutical composition contains a compound of any of Formulas I, I-a, I-b, and I-c or a pharmaceutically acceptable salt thereof and a pharmaceutically acceptable adjuvant.

The pharmaceutically acceptable adjuvant includes one or more of an excipient, a surfactant, a PH regulator, an osmotic pressure regulator, a lubricant, a disintegrant, a stabilizer, a suspending agent, a flavoring agent, a solvent, an emulsifier, a dispersant, and a solubilizer.

The pharmaceutical composition can be administered by inhalation in the form of a solution, a suspension, an aerosol, and a dry powder, etc; administered by infusion in the form of a reconstituted freeze-dried powder; administered by injection in the form of an injectable solution; or administered by orally taking in the form of a tablet, a capsule, or granules.

The compound of the present invention can inhibit the activity of neutrophil elastase (NE), and is thus an NE inhibitor that can be used in the prevention or treatment of diseases caused by abnormal increase of NE.

In another aspect of the present invention, use of the compound of any of Formulas I, I-a, I-b, and I-c or the pharmaceutically acceptable salt thereof or the pharmaceutical composition thereof in the preparation of a drug for treating or preventing diseases caused by abnormal increase of NE.

The diseases include autoimmune inflammation, infectious inflammation, traumatic inflammation, allergic inflammation, systemic inflammation, respiratory inflammation, lung inflammation and pain.

The diseases include inflammatory diseases, including ALI, ARDS, and ALI/ARDS with SIRS, etc.

### DETAILED DESCRIPTION

### Definitions of terms

"Halogen" includes fluorine, chlorine, bromine, iodine.

"Alkyl" refers to a saturated aliphatic hydrocarbon group, which may be a linear or branched alkyl group, preferably C₁-C₆ alkyl, and more preferably C₁-C₃ alkyl. Non-limiting examples include methyl, ethyl, n-propyl, isopropyl, or n-butyl, isobutyl, t-butyl, n-pentyl, n-hexyl and the like. The alkyl may be unsubstituted or substituted. When substitution exists, the substituent can be at any position where substitution is suitable.

"Alkoxy" is -O-alkyl or -O-cycloalkyl. Non-limiting examples of -O-alkyl include: methoxy, ethoxy, n-propoxy, isopropoxy, n-butoxy, isobutoxy, and tert-butoxy, etc. Non-limiting examples of -O-cycloalkyl include cyclopropoxy, cyclobutoxy, cyclopentyloxy, cyclohexyloxy, and that like. The alkoxy may be further substituted on the alkyl or cycloalkyl.

"Cycloalkyl" refers to a saturated or partially saturated monocyclic or polycyclic hydrocarbon substituent, preferably C₃-C₆ cycloalkyl. Non-limiting examples include cyclopropyl, cyclobutyl, cyclopentyl, cyclopentenyl, cyclohexyl, cyclohexenyl and the like. The cycloalkyl may be unsubstituted or further substituted with a substituent. When substituted, the substituent may be at any position on the ring where substitution is suitable.

"Heterocycloalkyl" refers to a saturated or partially saturated monocyclic or polycyclic hydrocarbon group containing 3 to 10 ring atoms, in which one or more ring atoms are selected from nitrogen, oxygen or sulfur. Preferably, the heterocycloalkyl is C₃-C₆ heterocycloalkyl. Non-limiting examples include oxetanyl, azetidinyl, and oxiranyl, etc. The heterocycloalkyl may be unsubstituted or further substituted with a substituent. When substituted, the substituent may be at any position on the ring where substitution is suitable.

"Heteroaryl" refers to a 5- to 10-membered heteroaromatic group containing 1 to 4 heteroatoms. The heteroatom includes nitrogen, oxygen and sulfur. Non-limiting examples include imidazolyl, pyrazolyl, oxazolyl, isoxazolyl, pyridinyl, pyrimidinyl, pyrazinyl, and the like. The heteroaryl may be attached to a ring carbon atom or to a heteroatom. The heteroaryl may be unsubstituted or further substituted with a substituent. When substituted, the substituent may be at any position on the ring where substitution is suitable.

"Aryl" refers to a 6- to 10-membered full carbon monocyclic or fused polycyclic ring with a conjugated π electron system, such as phenyl and naphthyl. The aryl can also be fused with heteroaryl, heterocycloalkyl, or cycloalkyl. The aryl may be unsubstituted or further substituted with a substituent.

When the alkyl, alkoxy, cycloalkyl, heterocycloalkyl, or heteroaryl in the present invention is substituted, it is preferably substituted with one or more of deuterium, C₁-C₃ alkyl, C₁-C₃ alkoxy, C₁-C₃ deuterated alkoxy, halogen, cyano, trifluoromethyl, nitro, amino, oxo (=O), -CONH₂, and -COOH.

"Oxo" refer to the substituent =O.

"Pharmaceutically acceptable salt" include alkali metal salts and salts formed with organic bases, such as a sodium salt, a potassium salt, and an amine salt, and also include salt hydrates.

In some embodiments of the present invention, the compound of the present invention is prepared by the following route 1: reacting Formula M1 with Formula M2 under a basic condition, and then removing the protecting group Pg to obtain the compound, 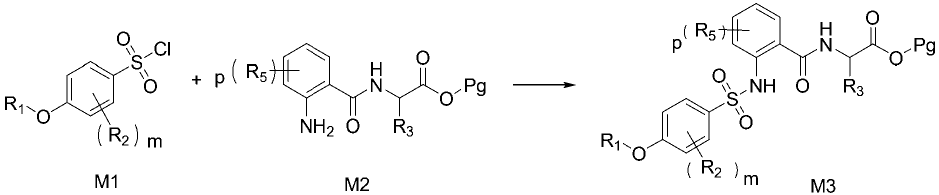 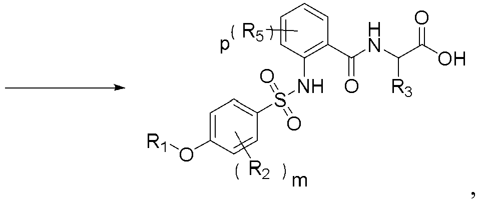 where R₁, R₂, R₃, R₅ and m, and p have the same definitions as in Formula I, and Pg is a protecting group, such as benzyl, and tert-butyl, etc.

In some embodiments of the present invention, the compound of the present invention is prepared by the following route 2: reacting a carboxylic acid compound substituted with R₁ with M4 in the presence of a condensing agent, and then removing the protecting group Pg to obtain the compound, 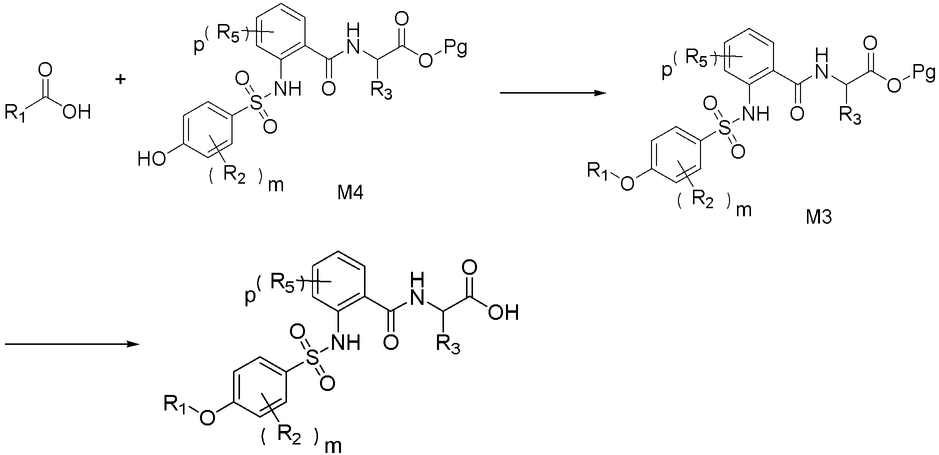 where R₁, R₂, R₃, R₅, m, and p have the same definitions as in Formula I, and Pg is a protecting group, such as benzyl, and tert-butyl, etc.

In some embodiments of the present invention, the compound of the present invention is prepared by the following route 3: 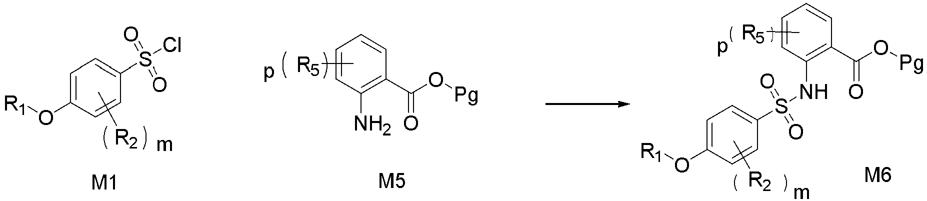 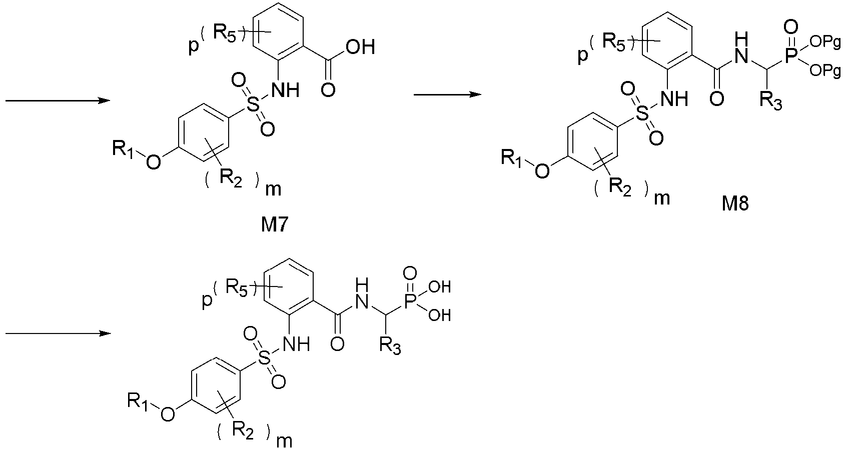
or reacting Formula M7 with formula 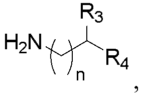
where R₁, R₂, R₃, R₅, m, and p have the same definitions as in Formula I, and Pg is a protecting group, such as benzyl, and tert-butyl, etc.

In a specific embodiment of the present invention, when ring A of the compound is a non-benzene ring, the compound can be prepared by a similar method with reference to Route 1 to Route 3.

The following examples are used to further illustrate the inventive concept of the present invention, and should not be construed as limitations on the present invention. Unless otherwise specified, the reagents or intermediates used in this patent can be commercially available or prepared by methods known in the art. Unless otherwise specified, the related drug preparation, biological activity test, detection of various indicators and establishment of animal models in the biological activity tests and animal tests of this patent can be carried out by conventional methods in the art.

### Preparation of intermediates

### Preparation of Intermediate 1

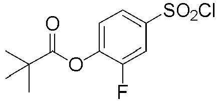

### Step 1: 4-(phenylmethylthio)-2-fluorophenol

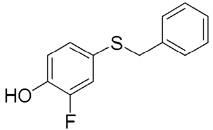

A mixture of 4-Bromo-2-fluorophenol (2 g, 10.5 mmol), phenylmethyl thiol (2 g, 15.8 mmoL), DIPEA (2.7 g, 21 mmoL), Pd₂dba₃ (916 mg, 1 mmoL) and Xant-phos (991 mg, 2.1 mmol) in dioxane (20 mL) was stirred overnight under an argon atmosphere at 110°C. The reaction mixture was evaporated and the residue was purified by column chromatography on silica gel (25% EtOAc/PE), to obtain 4-(phenylmethylthio)-2-fluorophenol (1 g, yield 42%).

MS (ESI): m/z = 235.1 [M+H]⁺.

### Step 2: 4-(phenylmethylthio)-2-fluorophenyl pivalate

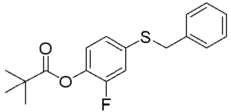

A mixture of 4-(phenylmethylthio)-2-fluorophenol (1 g, 4.3 mmol), pivaloyl chloride (564 mg, 4.7 mmol) and pyridine (2.7 g, 34.4 mmol) in DCM (10 mL) was stirred at room temperature for 3 hrs. H₂O (2 mL) was added to the reaction mixture. The aqueous phase was separated and extracted with EtOAc (5 mL x 3). The combined organic layer was dried over anhydrous Na₂SO₄, filtered and evaporated. The residue was purified by column chromatography on silica gel (10-60% EtOAc/petroleum ether), to obtain 4-(phenylmethylthio)-2-fluorophenyl pivalate (700 mg, yield 51%). MS (ESI): m/z = 319.1 [M+H]⁺.

### Step 3: 4-(chlorosulfonyl)-2-fluorophenyl pivalate

A solution of 4-(phenylmethylthio)-2-fluorophenyl pivalate (450 mg, 1.52 mmol)/1,3-dichloro-5,5-dimethylimidazolidin-2,4-dione (600 mg, 3.05 mmol)/ AcOH (0.8 mL)/ H₂O (0.5 mL) in CH₃CN (12 mL) was stirred at room temperature for 15 min. EA (100 mL) was added to the reaction mixture, followed by washing with H₂O (50 mL) and brine (50 mL). The organic layer was dried over Na₂SO₄, filtered and evaporated. The residue was purified by column chromatography on silica gel (10-60% EtOAc/petroleum ether), to obtain 4-(chlorosulfonyl)-2-fluorophenyl pivalate as an orange/brow solid (290 mg, yield 69%). MS (ESI): m/z = 295.0 [M+H]⁺.

### Preparation of Intermediate 2

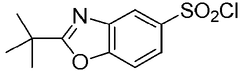

### Step 1: 4-bromo-2-pivalamidophenyl pivalate

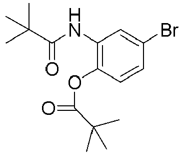

At 0°C, pivaloyl chloride (1.5 mL, 12.2 mmol) was added dropwise to a solution of 2-amino-4-bromophenol (2.5 g, 13.3 mmol)/DIPEA (3.3 mL, 20 mmol) in DCM (50 mL). The reaction mixture was then stirred at room temperature for 2 hrs. The reaction mixture was evaporated. The residue was purified by SGC (0-20% EA/PE, Rf = 0.7 in PE/EA 4/1), to obtain 4-bromo-2-pivalamidophenyl pivalate as a light brow solid (1.6 g, yield 33%).

MS (ESI): m/z = 356.2 [M+H]⁺.

### Step 2: 5-bromo-2-(t-butyl)benzo[d]oxazole

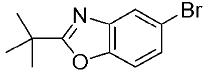

A mixture of 4-bromo-2-pivalamidophenyl ester (1.6 g, 4.5 mmol)/ p-TsOH H₂O (1.7 g, 8.9 mmol) in toluene (80 mL) was refluxed for 1.5 hrs in the Dean-Stark evaporator system at 130°C. The reaction mixture was cooled to room temperature and evaporated. The residue was purified by SGC (0-20% EA/PE, 0-10% MeOH/DCM, Rf = 0.4 in PE/EA 6/1), to obtain 5-bromo-2-(t-butyl)benzo[d]oxazole (0.58 g, yield 50%). MS (ESI): m/z = 255.9 [M+H]⁺.

### Step 3: 5-(phenylmethylthio)-2-(t-butyl)benzo[d]oxazole

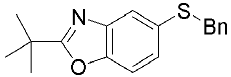

A mixture of 5-bromo-2-(t-butyl)benzo[d]oxazole (550 mg, 2.17 mmol)/phenylmethyl thiol (280 µL, 2.39 mmol)/ DIPEA (720 µL, 4.4 mmol)/ Pd₂dba₃ (200 mg, 0.22 mmol)/ Xantphos (253 mg, 0.44 mmol) in toluene (10 mL) was stirred overnight under a Ar atmosphere at 110°C. The reaction mixture was evaporated. The residue was purified by SGC (0-10% EA/PE, Rf = 0.6 in PE/EA 8/1), to obtain 5-(phenylmethylthio)-2-(t-butyl)benzo[d]oxazole as a yellow oil (0.6 g, yield 93%). MS (ESI): m/z = 298.2 [M+H]⁺.

### Step 4: 2-(t-butyl)benzo[d]oxazole -5-sulfonyl chloride

A solution of 5-(phenylmethylthio)-2-(t-butyl)benzo[d]oxazole (450 mg, 1.52 mmol)/1,3-dichloro-5,5-dimethylimidazolidin-2,4-dione (600 mg, 3.05 mmol)/ AcOH (0.8 mL)/ H₂O (0.5 mL) in CH₃CN (12 mL) was stirred at room temperature for 15 min. The reaction mixture was dissolved in EA (100 mL), and washed with H₂O (50 mL) and brine (50 mL). The EA layer was dried over Na₂SO₄, filtered and evaporated. The residue was purified by SGC (PE:EA = 10:1, Rf = 0.6 in PE/EA 10/1), to obtain 2-(t-butyl)benzo[d]oxazole -5-sulfonyl chloride as an orange/brown solid (290 mg, yield 69%). MS (ESI): m/z = 274.1 [M+H]⁺.

### Preparation of Intermediate 3

### (2-Aminobenzoyl)glycine benzyl ester

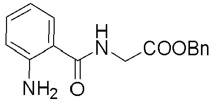

A mixture of 2H-benzo[d][1,3]oxazin-2,4(1H)-dione (5 g, 30.7 mmol), glycine benzyl ester hydrochloride (6.8 g, 33.7 mmol), and TEA (10.5 mL, 75.5 mmol) in CH₃CN (100 mL) was stirred at 70°C for 4 hrs. The reaction mixture was cooled to room temperature and evaporated. The residue (grey solid/oil) was added with water (100 mL) and extracted with EA (500 mL, 100 mL x 2). The EA layers were combined, washed with brine (100 mL), dried over Na₂SO₄, filtered and evaporated. The residue was washed with EA (50 mL), to obtain (2-aminobenzoyl)glycine benzyl ester as a white solid (2.39 g, yield 27%). MS (ESI): m/z = 285.1 [M+H]⁺.

### Preparation of Intermediate 4

### (4-amino-1-methyl-1H-pyrazol-3-carbonyl)glycine benzyl ester

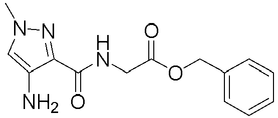

### Step 1: 1-methyl-4-nitro-1H-pyrazol-3-carboxylic acid

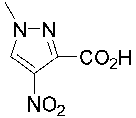

A mixture of methyl 1-methyl-4-nitro-1H-pyrazol-3-carboxylate (1800 mg, 9.72 mmol) and LiOH·H₂O (815 mg, 19.44 mmol) in THF (10 mL) and H₂O (10 mL) was stirred at room temperature for 2 hrs. The reaction mixture was adjusted to pH 4-5 with Amberlyst 15 (H) ion exchange resin. The reaction mixture was filtered and the filtrate was evaporated, to obtain 1-methyl-4-nitro-1H-pyrazol-3-carboxylic acid as a yellow solid (1.6 g, yield 96%). MS (ESI) m/z = 172.1 [M+H]⁺.

### Step 2: (1-methyl-4-nitro-1H-pyrazol-3-carbonyl)glycine benzyl ester

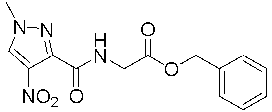

A mixture of 1-methyl-4-nitro-1H-pyrazol-3-carboxylic acid (1.6 g, 9.36 mmol), glycine benzyl ester hydrochloride (2.8 g, 14.04 mmol), HATU (4.3 g, 11.23 mmol), HOAt (1.5 g, 11.23 mmol) and TEA (4 mL, 28.80 mmol) in DMF (20 mL) was stirred at room temperature for 1 hr. The mixture was added with EA (100 mL) and washed with brine (3 x 100 mL). The EA layer was separated and evaporated. The residue was purified by column chromatography on silica gel (eluting with EA), to obtain (1-methyl-4-nitro-1H-pyrazol-3-carbonyl)glycine benzyl ester as a white solid (2.1 g, yield 70%). MS (ESI) m/z = 319.2 [M+H]⁺.

### Step 3: (4-amino-1-methyl-1H-pyrazol-3-carbonyl)glycine benzyl ester

A solution of (1-methyl-4-nitro-1H-pyrazol-3-carbonyl)glycine benzyl ester (2.1 g, 6.60 mmol) and Fe (2.2 g, 39.62 mmol) in MeOH (4 mL) and saturated NH₄Cl (20 mL) was stirred at 80°C for 2 hrs. The reaction mixture was filtered and the filter cake was washed with EA (100 mL) and H₂O (20 mL). The filtrate was neutralized to pH 7 with saturated NaHCO₃. The EA layer was separated and evaporated. The residue was purified by SGC (EA=100%, Rf = 0.4 in EA), to obtain (4-amino-1-methyl-1H-pyrazol-3-carbonyl)glycine benzyl ester as a white solid (1 g, yield 52%). MS (ESI) m/z = 289.3 [M+H]⁺.

### Preparation of Intermediate 5

### 2-((4-(pivaloyloxy)phenyl)sulfonamido)benzoic acid

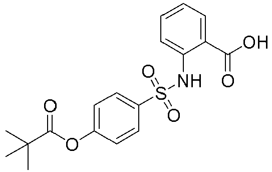

### Step 1: Benzyl 2-((4-(pivaloyloxy)phenyl)sulfonamido)benzoate

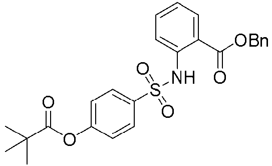

To a solution of benzyl 2-amino-benzoate (500 mg, 2.2 mmol) and pyridine (2 mL) in DCM (15 mL), 4-(pivaloyloxy)phenyl)sulfonyl chloride (670 mg, 2.4 mmol) was added. The obtained mixture was stirred overnight at room temperature. The reaction mixture was concentrated and the residue was purified by column chromatography on silica gel (eluting with PE/EtOAc = 4/1, V/V), to obtain a product as a white solid (1 g, yield 97%). MS (ESI): m/z = 468.1 [M+H]⁺.

### Step 2: 2-((4-(pivaloyloxy)phenyl)sulfonamido)benzoic acid

Benzyl 2-((4-(pivaloyloxy)phenyl)sulfonamido)benzoate (1 g, 2.1 mmol) and Pd/C (250 mg, 10% on C) were mixed with THF (30 mL) and added to a hydrogenation device. The system was evacuated and then refilled with hydrogen 4 times. The mixture was stirred at room temperature for 3 hrs. The mixture was filtered and the filtrate was concentrated, to obtain a crude product as a white solid (690 mg, yield 90%). MS (ESI): m/z = 378.1 [M+H]⁺.

### Preparation of Intermediate 6

### (2-amino-5-fluorobenzoyl)-D-alanine tert-butyl ester

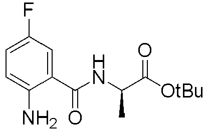

A mixture of 6-fluoro-2H-benzo[d][1,3]oxazin-2,4(1H)-dione (1 g, 5.5 mmol), D-alanine tert-butyl ester hydrochloride (880 mg, 6 mmol) and TEA (1.6 g, 16.5 mmol) in THF (10 mL) was refluxed for 3 hrs. The reaction mixture was quenched with H₂O (2 mL) and extracted with EtOAc (5 mL x 3). The combined organic layer was washed with brine (10 mL), dried over anhydrous Na₂SO₄, filtered and concentrated. The residue was purified by column chromatography on silica gel (10-60% EtOAc/petroleum ether), to obtain (2-amino-5-fluorobenzoyl)-D-alanine tert-butyl ester (850 mg, yield: 89%). MS (ESI): m/z = 283.1 [M+H]⁺.

### Preparation of Intermediate 7

### (2-amino-5-cyanobenzoyl)-D-alanine tert-butyl ester

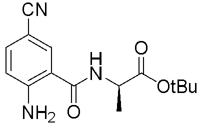

A mixture of 2-amino-5-cyanobenzoic acid (1 g, 6 mmol), D-alanine tert-butyl ester hydrochloride (1.2 g, 6.6 mmol), HOBt (891 mg, 6.6 mmol), EDCI HCl (1 g, 6.6 mmol) and DIPEA (2.3 g, 18 mmol) in DCM (10 mL) was stirred at room temperature for 3 hrs. H₂O (2 mL) was added to the reaction mixture. The aqueous phase was separated and extracted with EtOAc (5 mL x 3). The combined organic layer was dried over anhydrous Na₂SO₄, filtered and concentrated. The residue was purified by column chromatography on silica gel (10-60% EtOAc/petroleum ether), to obtain (2-amino-5-cyanobenzoyl)-D-alanine tert-butyl ester (1.3 g, yield 80%). MS (ESI): m/z = 312.2 [M+Na]⁺.

### Preparation of Intermediate 8

### (2-amino-5-methoxybenzoyl)glycine benzyl ester

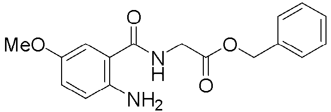

A mixture of 2-amino-5-methoxybenzoic acid (500 mg, 3 mmol), glycine benzyl ester hydrochloride (600 mg, 3 mmol), EDCI HCl (634 mg, 3.3 mmol), HOBt (446 mg, 3.3 mmol) and DIPEA (1.2 g, 9 mmol) in DCM (10 mL) was stirred overnight at room temperature. The mixture was quenched with H₂O (10 mL), and extracted with DCM (30 mL x 3). The organic phases were combined, washed with brine, dried over anhydrous Na₂SO₄, filtered and concentrated. The residue was purified by column chromatography on silica gel (15% EtOAc/PE), to obtain (2-amino-5-methoxybenzoyl)glycine benzyl ester as a yellow oil (800 mg, yield 85%). MS (ESI): m/z 315.1 [M+H]⁺.

### Preparation of Intermediate 9

### Benzyl (S)-2-amino-3-fluoropropionate 2,2,2-trifluoroacetate

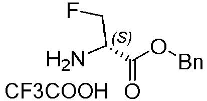

### Step 1: 4-Benzyl 3-(t-butyl) (R)-1,2,3-oxathiazolidine-3,4-dicarboxylate 2,2-dioxide

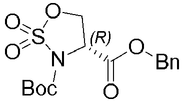

Under Ar at -40°C, a solution of (t-butoxycarbonyl)-D-serine benzyl ester (5 g, 16.9 mmol) in DCM (170 mL) was added dropwise to a solution of SOCl₂ (3.1 mL, 42.7 mmol) in DCM (85 mL). The temperature was maintained to be <-35°C. Then, the reaction mixture was stirred for 30 min at -35°C to -40°C. Py (7.1 mL, 87.9 mmol) was slowly added to the reaction mixture. The reaction mixture was heated to room temperature and stirred for 1 hr. The reaction mixture was quenched with iced water (110 mL), washed with saturated NaHCO₃ (110 mL), dried over Na₂SO₄, filtered and evaporated, to obtain an intermediate as a colorless oil (7 g). In a solution at 0°C, NaIO₄ (6 g, 28 mmol) in water (110 mL) was added dropwise to the solution of the intermediate (7 g)/ RuCl₃ (80 mg, 0.39 mmol) in CH₃CN (110 mL). The reaction mixture was then stirred at 0°C for 1 hr. The reaction mixture was quenched with saturated NaHCO₃ (110 mL) and extracted with EA (150 mL x 3). The organic layers were combined, washed with brine (150 mL), filtered and evaporated. The residue was purified by SGC (0-25% EA/PE, Rf = 0.4 in PE/EA 4/1, weak UV, ninhydrin), to obtain a compound as a white solid (4.2 g, yield 69%). MS (ESI): m/z = 380.3 [M+Na]⁺.

### Step 2: Benzyl (S)-2-((t-butoxycarbonyl)amino)-3-fluoropropionate

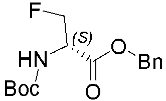

KF (3.9 g, 10.9 mmol) and 18-crown ether-6 (1.9 g, 32.8 mmol) were suspended in anhydrous CH₃CN (100 mL) and stirred at room temperate for 15 min. Then, the compound of Step 1 (3.9 g, 10.9 mmol) was added to the reaction mixture, and the mixture was stirred overnight at room temperature. The reaction mixture was evaporated. DCM (60 mL)/ 20% H₂SO₄ (20 mL) was added to the residue and stirred at room temperature for 1.5 hrs. The reaction mixture was neutralized to pH 7 with saturated NaHCO₃ and extracted with DCM (100 mL x 2). The DCM layers were combined, dried over Na₂SO₄, filtered and evaporated. The residue was purified by SGC (0-25% EA/PE, Rf of the product = 0.5 in PE/EA 4/1), to obtain a compound as a colorless oil (130 mg, yield 4%). MS (ESI): m/z = 320.2 [M+Na]⁺.

### Step 3: Benzyl (S)-2-amino-3-fluoropropionate 2,2,2-trifluoroacetate

A mixture of the compound of Step 2 (130 mg, 0.44 mmol) in TFA (1 mL) / DCM (4 mL) was stirred at room temperature for 1.5 hrs. DCM (20 mL) was added to the reaction mixture and evaporated to dryness. The residue was dissolved in DCM (20 mL) and evaporated to dryness again, to obtain the crude product benzyl (S)-2-amino-3-fluoropropionate 2,2,2-trifluoroacetate as a colorless oil (200 mg). This crude product was directly used in the next step. MS (ESI): m/z = 198.2 [M+H]⁺.

### Preparation of Intermediate 10

### N-(2-aminobenzoyl)-O-methyl-D-serine benzyl ester

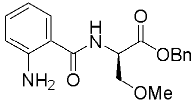

### Step 1: N-(t-butoxycarbonyl)-O-methyl-D-serine benzyl ester

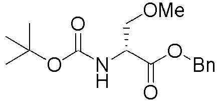

A mixture of N-(t-butoxycarbonyl)-O-methyl-D-serine (1 g, 4.6 mmol), CbzCl (1.56 g, 9.2 mmol), TEA (0.9 g, 9.2 mmol) and DMAP (112 mg, 0.92 mmol) in DCM (10 mL) was stirred overnight at 0°C. The mixture was quenched with H₂O (10 mL) and extracted with DCM (30 mL x 3). The organic phases were combined, washed with brine, dried over anhydrous Na₂SO₄, filtered and concentrated. The residue was purified by column chromatography on silica gel (13% EtOAc/PE), to obtain the product N-(t-butoxycarbonyl)-O-methyl-D-serine benzyl ester as a colorless oil (1.3 g, yield 92%). MS (ESI): m/z 210.1 [M-99]⁺.

### Step 2: O-methyl-D-serine benzyl ester

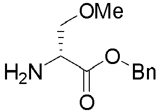

A mixture of N-(t-butoxycarbonyl)-O-methyl-D-serine benzyl ester (1.3 g, 4.2 mmol) and TFA (5 mL) in DCM (50 mL) was stirred at room temperature for 2 hrs. The mixture was dried under vacuum, to obtain a product as a white solid (800 mg, crude material). This crude product was directly used in the next step. MS (ESI): m/z = 210.2 [M+H]⁺.

### Step 3: N-(2-aminobenzoyl)-O-methyl-D-serine benzyl ester

A mixture of the product of Step 2 (200 mg, 0.62 mmol), 2H-benzo[d][1,3]oxazin-2,4(1H)-dione (186 mg, 1.14 mmol) and TEA (192 mg, 1.9 mmol) in THF (5 mL) was stirred overnight at room temperature. The reaction mixture was concentrated and purified by column chromatography on silica gel (25% EtOAc/PE), to obtain *N*-(2-aminobenzoyl)-*O*-methyl-*D*-serine benzyl ester as a colorless oil (100 mg, yield 49%). MS (ESI): m/z 329.1 [M+H]⁺.

### Preparation of Intermediate 11

### ((1R,2R)-2-amino-5,5-difluorocyclohexan-1-carbonyl)glycine t-butyl ester

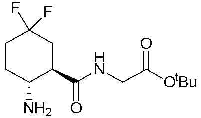

### Step 1: ((1R,2R)-2-(((benzyloxy)carbonyl)amino) -5,5-difluorocyclohexan -1-carbonyl)glycine t-butyl ester

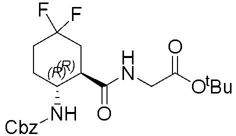

A mixture of (1R,2R)-2-(((benzyloxy)carbonyl)amino)-5,5-difluorocyclohexan-1-carboxylic acid (900 mg, 2.88 mmol), HOBt (388 mg, 2.88 mmol), DIPEA (1.11 g, 8.63 mmol) and HBTU (1.42 g, 3.74 mmol) in THF (30 mL) was stirred at room temperature for 5 min. Then, glycine t-butyl ester (565 mg, 4.31 mmol) was added and the mixture was stirred at room temperature for 2 hrs. The reaction mixture was added with water (100 mL) and extracted with EtOAc (200 mL x 3). The organic layers were combined, washed with brine (150 mL), dried over Na₂SO₄, filtered, and concentrated. The residue was purified by column chromatography on silica gel (eluting with EtOAc/PE = 1/3 to 1/2, V/V), to obtain a product as a white solid (1 g, yield 82%). MS (ESI): m/z = 449.2 [M+Na]⁺.

### Step 2: ((1R,2R)-2-amino-5,5-difluorocyclohexan-1-carbonyl)glycine t-butyl ester

### The compound of Step 1 (1 g, 2.3 mmol) and Pd/C (250 mg, 10% on C) were mixed with THF (30 mL) and added to a hydrogenation device. The system was evacuated and then refilled with hydrogen 4 times. The mixture was stirred overnight at room temperature. The mixture was filtered and the filtrate was concentrated, to obtain a crude product as a white solid (690 mg, yield 90%). MS (ESI): m/z = 293.1 [M+H]⁺.

### Example 1

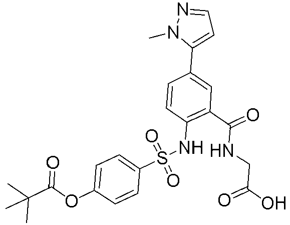

### Step 1: (2-amino-5-bromobenzoyl)glycine benzyl ester

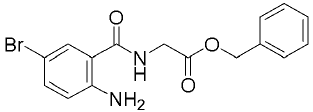

To a solution of 6-bromo-2H-benzo[d][1,3]oxazin-2,4(1H)-dione (2 g, 8.26 mmol) in MeCN (50 mL), glycine benzyl ester hydrochloride (1.83 g, 9.08 mmol) and TEA (1.25 g, 12.39 mmol) were added. The mixture was heated to 60°C, stirred for 16 hrs, and monitored by LCMS. The obtained mixture was extracted with EA (30 mL x 3). The organic layer was washed with brine, dried over Na₂SO₄, filtered and concentrated under vacuum. The residue was purified by column chromatography on silica gel eluting with PE/EA (1/9), to obtain (2-amino-5-bromobenzoyl)glycine benzyl ester as a brown solid (2.3 g, yield 76%). MS (ESI): m/z = 365.0 [M+H]⁺.

### Step 2: (2-amino-5-(1-methyl-1H-pyrazol-5-yl)benzoyl)glycine benzyl ester

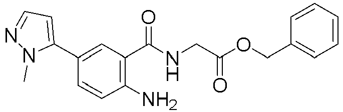

A mixture of (2-amino-5-bromobenzoyl)glycine benzyl ester (600 mg, 1.65 mmol), 1-methyl-5-(4,4,5,5-tetramethyl-1,3,2-dioxaborolan-2-yl)-1H-pyrazole (412 mg, 1.98 mmol), Pd(dppf)Cl₂ (60 mg, 0.08 mmol) and potassium acetate (485 mg, 4.95 mmol) in dioxane /H₂O (30 mL, 5:1) was stirred under Ar at 80°C for 16 hrs. The reaction was monitored by LCMS. The obtained mixture was extracted with EA (30 mL x 3). The organic layers were combined, washed with brine, dried over Na₂SO₄, filtered and concentrated under vacuum. The residue was purified by SGC (0-30% EA/PE, Rf of the product = 0.5 in PE/EA 2/1), to obtain (2-amino-5-(1-methyl-1H-pyrazol-5-yl)benzoyl)glycine benzyl ester as a light brown gel (340 mg, yield 68%). MS (ESI): m/z = 365.1 [M+H]⁺.

### Step 3: 4-(N-(2-((2-(benzyloxy)-2-oxoethyl)carbamoyl)-4-(1-methyl-1H-pyrazol-5-yl)phenyl)aminos ulfonyl)phenyl pivalate

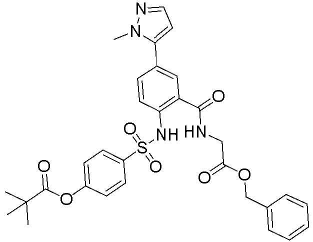

To a solution of (2-amino-5-(1-methyl-1H-pyrazol-5-yl)benzoyl)glycine benzyl ester (350 mg, 0.96 mmol) in DCM (20 mL), 4-(chlorosulfonyl)phenyl pivalate (397 mg, 1.44 mmol) and Py (1137 mg, 14.4 mmol) were added. The mixture was stirred at room temperature for 16 hrs. The reaction mixture was added with water (30 mL) and extracted with DCM (30 mL x 3). The organic layers were combined, washed with brine (20 mL), dried over anhydrous Na₂SO₄, filtered and evaporated. The residue was purified by column chromatography on silica gel (EtOAC/petroleum ether, 0% to 80%), to obtain a brown gel (240 mg, yield 41%). MS (ESI): m/z = 605.1 [M+H]⁺.

### Step 4: (5-(1-methyl-1H-pyrazol-5-yl)-2-((4-(pivaloyloxy)phenyl)sulfonamido)benzoyl)glycine

At room temperature, to a suspension of the compound of Step 3 (200 mg, 0.33 mmol) in THF (30 mL), 10% Pd/C (50 mg) was added. The mixture was stirred overnight at room temperature under H₂ atmosphere. The reaction was monitored by LCMS. Then, the mixture was filtered, and the filtrate was concentrated, to obtain a crude product. The crude product was purified by preparative HPLC (NH₄HCO₃ buffer: A: 10 mM NH₄HCO₃/water; B: acetonitrile; column: Waters XBridge Peptide BEH C18, 19 x 250 mm, 10 µm, 130 Å), to obtain a target compound as a white solid (138 mg, yield 73%).

MS (ESI) m/z: 515.3 [M+H]⁺. LCMS purity: 100.00% (214 nm), RT = 1.749 min.

¹H NMR (400 MHz, DMSO-*d₆*): δ 12.81 (s, 1H), 11.72 (s, 1H), 9.35 (s, 1H), 7.91-7.80 (m, 3H), 7.67 (d, *J* = 8 Hz, 1H), 7.60 (d, *J* = 8 Hz, 1H), 7.46 (d, *J* = 1.6 Hz, 1H), 7.32 (d, *J* = 8 Hz, 2H), 6.42 (d, *J* = 2.0 Hz, 1H), δ 3.94 (d, *J* = 5.6 Hz, 2H), 3.80 (s, 3H), 1.27 (s, 9H).

Using a method similar to that in Example 1, the following compounds were prepared with different intermediates.

| Examples | Compound structure | ¹H-NMR, MS |
|---|---|---|
| 2 | 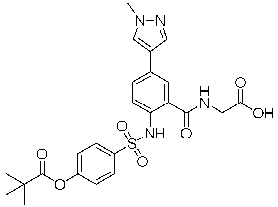 | MS (ESI) m/z: 515.3 [M+H] ⁺ |
| 3 | 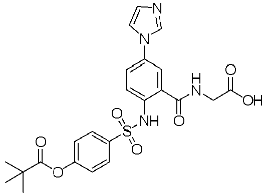 | MS (ESI) m/z = 501.3 [M+H] ⁺. |
| | | ¹H NMR (400 MHz, DMSO-d₆): δ 12.9 (brs, 1H), 11.6 (brs, 1H), 9.38 (t, *J*= 5.6 Hz, 1H), 9.21 (s, 1H), 8.12-8.07 (m, 2H), 7.91-7.87 (m, 3H), 7.70-7.67 (m, 2H), 7.35-7.32 (m, 2H), 4.00 (d, *J*= 5.6 Hz, 2H), 1.27 (s, 9H). |
| 4 | 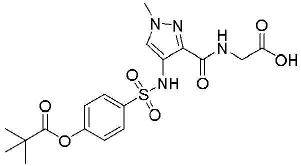 | MS (ESI) m/z = 439.2 [M+H] ⁺. |
| | | ¹H NMR (400 MHz, DMSO-d₆): δ 12.6 (brs, 1H), 9.27 (s, 1H), 8.35 (t, *J* = 5.6 Hz, 1H), 7.86-7.81 (m, 3H), 7.30-7.28 (m, 2H), 3.83 (s, 3H), 3.80 (d, *J* = 6 Hz, 2H), 1.29 (s, 9H). |
| 5 | 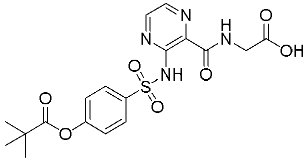 | MS (ESI) m/z = 437.1 [M+H] ⁺. |
| | | ¹H NMR (400 MHz, DMSO-d₆): δ 12.7 (brs, 1H), 11.9 (brs, 1H), 9.52 (s, 1H), 8.49 (d, *J* = 2 Hz, 1H), 8.37 (d, *J* = 2 Hz, 1H), 8.13 (d, *J* = 8.8 Hz, 2H), 7.35 (d, *J* = 8.8 Hz, 2H), 3.98 (d, *J* = 6 Hz*,* 2H), 1.29 (s, 9H). |
| 6 | 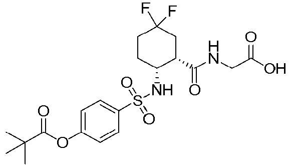 | MS (ESI): m/z = 477.3 [M+H]⁺. |
| | | ¹H NMR (400 MHz, DMSO-d₆): δ 12.55 (s, 1 H), 8.13 (t, *J* = 5.4 Hz, 1 H), 7.84 (d, *J =* 8.4 Hz, 1 H), 7.80 (d, *J* = 8.4 Hz, 2 H), 7.20 (d, *J* = 8.8 Hz, 2 H), 3.83-3.81 (m, 1 H), 3.75-3.69 (m, 1 H), 3.37-3.31 (m, 1 H), 2.70-2.67 (m, 1 H), 2.41-2.24 (m, 1 H), 2.06-1.77 (m, 3 H), 1.54-1.47 (m, 2 H), 1.31 (s, 9 H). |
| 7 | 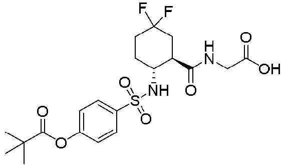 | MS (ESI): m/z = 477.3 [M+H]⁺. |
| | | ¹H NMR (400 MHz, DMSO-d₆): δ 12.56 (s, 1 H), 8.08 (t, *J=* 5.8 Hz, 1 H), 7.78 (d, *J =* 8.8 Hz, 2 H), 7.70 (d, *J=* 8.8 Hz, 1 H), 7.27 (d, *J* = 8.4 Hz, 2 H), 3.71-3.65 (m, 1 H), 3.55-3.50 (m, 1 H), 3.49-3.43 (m, 1 H), 2.55-2.50 (m, 1 H), 2.07-1.88 (m, 4 H), 1.75-1.69 (m, 1 H), 1.48-1.37 (m, 1 H), 1.31 (s, 9 H). |
| 8 | 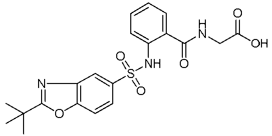 | MS (ESI): m/z = 432.2 [M+H]⁺. |
| | | ¹H NMR (400 MHz, DMSO-d₆): δ 12.7 (brs, 1H), 11.56 (s, 1H), 9.16-9.12 (m, 1H), 8.09 (d, *J* = 1.6 Hz, 1H), 7.86-7.83 (m, 1H), 7.77-7.74 (m, 1H), 7.71-7.68 (m, 1H), 7.58-7.56 (m, 1H), 7.50-7.45 (m, 1H), 7.15-7.10 (m, 1H), 3.87 (d, *J* = 6 Hz, 2H), 1.41 (s, 9H) |
| 9 | 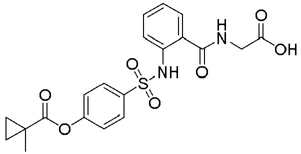 | MS (ESI): m/z = 433.2 [M+H]⁺. |
| | | ¹H NMR (400 MHz, DMSO-d₆): δ 12.6 (brs, 1H), 11.58 (s, 1H), 9.23-9.20 (m, 1H), 7.80-7.77 (m, 2H), 7.74-7.72 (m, 1H), 7.52-7.45 (m, 2H), 7.30-7.27 (m, 2H), 7.17-7.13 (m, 1H), 3.90 (d, *J* = 5.6 Hz, 2H), 1.32-1.29 (m, 5H), 0.93-0.90 (m, 2H). |
| 10 | 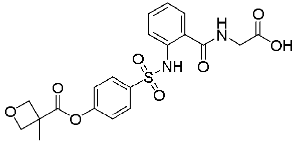 | MS (ESI): m/z = 449.1 [M+H]⁺. |
| | | ¹H NMR (400 MHz, DMSO-d₆): δ 12.7 (brs, 1H), 11.60 (s, 1H), 9.24-9.21 (m, 1H), 7.85-7.82 (m, 2H), 7.75-7.73 (m, 1H), 7.53-7.46 (m, 2H), 7.41-7.38 (m, 2H), 7.18-7.14 (m, 1H), 4.91 (d, *J* = 6 Hz, 2H), 4.40 (d, *J* = 6 Hz, 2H), 3.91 (d, *J* = 6 Hz, 2H), 1.63 (s, 3H). |
| 11 | 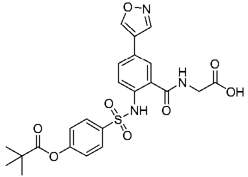 | MS (ESI): m/z = 502.2 [M+H]⁺. |
| | | ¹H NMR (400 MHz, DMSO-d₆): δ 12.7 (brs, 1H), 11.6 (brs, 1H), 9.50-9.20 (m, 2H), 9.06 (s, 1H), 8.08 (d, *J* = 2 Hz, 1H), 7.84-7.76 (m, 3H), 7.55 (d, *J* = 8.4 Hz, 1H), 7.28 (d, *J* = 8.8 Hz, 2H), 3.97 (d, *J* = 5.6 Hz, 2H), 1.26 (s, 9H) |
| 14 | 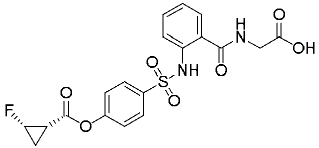 | MS (ESI): m/z = 437.1 [M+H]⁺. |
| | | ¹H NMR (400 MHz, DMSO-d₆): δ 12.7 (brs, 1H), 11.59 (s, 1H), 9.24-9.21 (m, 1H), 7.83-7.80 (m, 2H), 7.74 (d, *J* = 7.6 Hz, 1H), 7.54-7.46 (m, 2H), 7.33-7.30 (m, 2H), 7.18-7.14 (m, 1H), 5.22-5.17 (m, 0.5H), 5.05-5.01 (m, 0.5H), 3.90 (d, *J* = 5.6 Hz, 2H), 2.22-2.15 (m, 1H), 1.73-1.63 (m, 1H), 1.44-1.34 (m, 1H). |
| 15 | 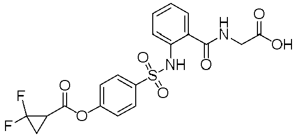 | MS (ESI): m/z = 455.1 [M+H]⁺. |
| | | ¹H NMR (400 MHz, DMSO-d₆): δ 12.8 (brs, 1H), 11.60 (s, 1H), 9.24-9.20 (m, 1H), 7.83 (d, *J* = 8.4 Hz, 2H), 7.74 (d, *J* = 7.6 Hz, 1H), 7.53-7.46 (m, 2H), 7.36 (d, *J* = 8.4 Hz, 2H), 7.18-7.14 (m, 1H), 3.90 (d, J = 6 Hz, 2H), 3.13-3.05 (m, 1H), 2.26-2.11 (m, 2H) |
| 16 | 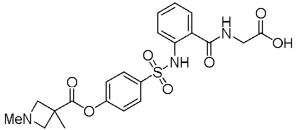 | MS (ESI): m/z = 462.3 [M+H]⁺. |
| 17 | 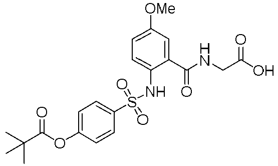 | MS (ESI): m/z =465.1 [M+H]⁺. |
| | | ¹H NMR (400 MHz, DMSO-d₆): δ 12.8 (brs, 1H), 11.00 (s, 1H), 9.14 (t, *J* = 5.8 Hz, 1H), 7.72-7.69 (m, 2H), 7.45 (d, *J* = 9.0 Hz, 1H), 7.28-7.23 (m, 3H), 7.10 (dd, *J* = 9.0, 2.9 Hz, 1H), 3.87 (d, *J* = 5.8 Hz, 2H), 3.74 (s, 3H), 1.28 (s, 9H). |
| 18 | 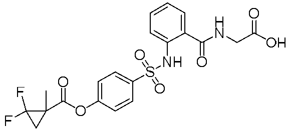 | MS (ESI): m/z = 469.2 [M+H]⁺. |
| | | ¹H NMR (400 MHz, DMSO-d₆): δ 12.7 (brs, 1H), 11.61 (s, 1H), 9.25-9.21 (m, 1H), 7.84-7.82 (m, 2H), 7.74 (d, *J* = 7.6 Hz, 1H), 7.52-7.46 (m, 2H), 7.35-7.33 (m, 2H), 7.18-7.14 (m, 1H), 3.90 (d, *J* = 6.4 Hz, 2H), 2.38-2.31 (m, 1H), 1.99-1.92 (m, 1H), 1.49 (s, 3H). |
| 19 | 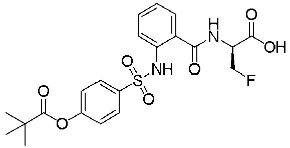 | MS (ESI): m/z = 467.2 [M+H]⁺. |
| | | ¹H NMR (400 MHz, DMSO-d₆): δ 13.29 (brs, 1H), 11.32 (brs, 1H), 9.34 (d, *J* = 6 Hz, 1H), 7.81-7.79 (m, 3H), 7.50-7.46 (m, 2H), 7.29-7.26 (m, 2H), 7.19-7.15 (m, 1H), 4.86-4.77 (m, 1H), 4.74-4.68 (m, 2H), 1.27 (m, 9H). |
| 20 | 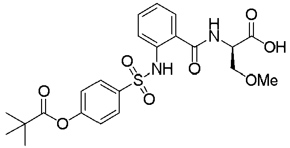 | MS (ESI): m/z =479.1 [M+H]⁺. |
| | | ¹H NMR (400 MHz, DMSO-d₆): δ 13.0 (brs, 1H), 11.39 (s, 1H), 9.05 (d, *J* = 7.3 Hz, 1H), 7.82-7.77 (m, 3H), 7.50-4.48 (m, 2H), 7.28 (dd, *J* = 7.2, 2.4 Hz, 2H), 7.21 - 7.12 (m, 1H), 4.62-4.58 (m, 1H), 3.77-3.66 (m, 2H), 3.29 (s, 3H), 1.27 (s, 9H). |
| 21 | 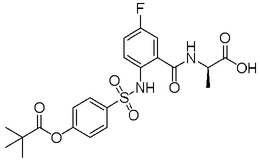 | MS (ESI): m/z = 467.1 [M+H]⁺. |
| | | ¹H NMR (400 MHz, DMSO-d₆): δ 12.9 (brs, 1H), 11.08 (s, 1H), 9.01 (d, *J* = 6.8 Hz, 1H), 7.76-7.72 (m, 2H), 7.62 (dd, *J* = 3.2, 9.6 Hz, 1H), 7.54-7.50 (m, 1H), 7.43-7.37 (m, 1H), 7.31-7.27 (m, 2H), 4.33-4.29 (m, 1H), 1.36 (d, *J* = 7.6 Hz, 3H), 1.28 (s, 9H). |
| 22 | 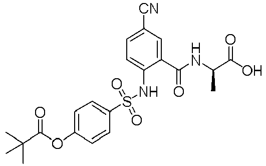 | MS (ESI): m/z = 474.1 [M+H]⁺. |
| | | ¹H NMR (400 MHz, DMSO-d₆): δ 12.8 (brs, 1H), 11.88 (s, 1H), 9.24 (d, *J* = 6 Hz, 1H), 8.31 (d, *J* = 1.6 Hz, 1H), 7.95-7.90 (m, 3H), 7.63 (d, *J* = 8.4 Hz, 1H), 7.36-7.33 (m, 2H), 4.41-4.37 (m, 1H), 1.40 (d, *J* = 7.6 Hz, 3H), 1.28 (s, 9H). |
| 23 | 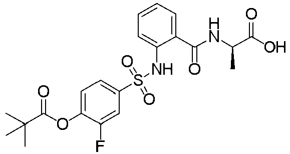 | MS (ESI): m/z = 467.1 [M+H]⁺. |
| | | ¹H NMR (400 MHz, DMSO-d₆): δ 12.8 (brs, 1H), 11.41 (s, 1H), 9.04 (d, *J* = 6.8 Hz, 1H), 7.79-7.75 (m, 2H), 7.65-7.62 (m, 1H), 7.51-7.47 (m, 3H), 7.23-7.18 (m, 1H), 4.36-4.32 (m, 1H), 1.37 (d, *J* = 7.2 Hz, 3H), 1.29 (s, 9H). |
| 24 | 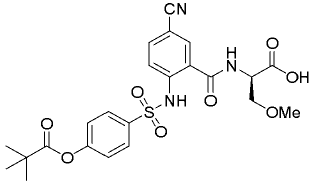 | MS (ESI): m/z = 504.6 [M+H]⁺. |
| | | 1H NMR (400 MHz, DMSO-d6) δ 12.60 (br, 1H), 11.62 (s, 1H), 8.80 (br, 1H), 8.15 (d, J = 2.1 Hz, 1H), 7.85 - 7.77 (m, 3H), 7.41 (t, J = 8.0 Hz, 1H), 7.13 (d, J = 8.4 Hz, 2H), 4.64 - 4.54 (m, 1H), 3.68 (dd, J = 9.8, 5.5 Hz, 1H), 3.57 (dd, J = 9.8, 4.2 Hz, 1H), 3.29 (s, 3H), 1.28 (s, 9H). |
| 26 | 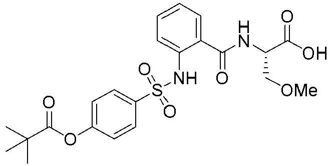 | MS (ESI): m/z =479.1 [M+H]⁺. |
| | | ¹H NMR (400 MHz, DMSO-d₆): δ 13.1 (brs, 1H), 11.28 (s, 1H), 9.03 (d, *J* = 7.3 Hz, 1H), 7.83-7.76(m, 3H), 7.51-4.47 (m, 2H), 7.29 (dd, *J* = 7.2, 2.4 Hz, 2H), 7.22 - 7.13 (m, 1H), 4.64-4.57 (m, 1H), 3.76-3.64 (m, 2H), 3.26 (s, 3H), 1.28 (s, 9H). |
| 27 | 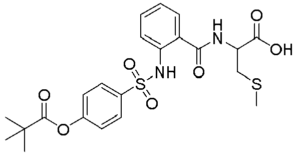 | MS (ESI): m/z =495.1 [M+H]⁺. |

### Example 12

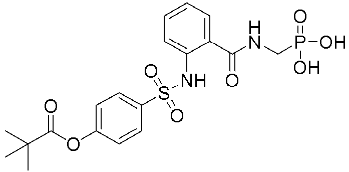

### Step 1: Diphenyl methyl ((triphenylmethylamino)methyl)phosphonate

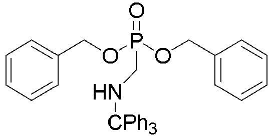

An aqueous formaldehyde solution (37%, 3.8 mL, 46.3 mmol) was added to a solution of triphenylmethyl amine (6 g, 23.2 mmol) in toluene (100 mL). The reaction mixture was stirred at room temperature for 20 hrs. Subsequently, water was removed by the Dean-Stark apparatus and a corresponding hydrogen phosphate (7.28 g, 27.8 mmol) and triethyl amine (234 mg, 2.3 mmol) were added. The reactant was stirred overnight in toluene under reflux. The mixture was concentrated and the residue was purified by column chromatography on silica gel (eluting with EtOAc/PE = 1/1, V/V), to obtain a product as a white solid (10 g, yield 82%). MS (ESI): m/z = 1089.2 [2M+Na]⁺.

### Step 2: Diphenyl methyl (aminomethyl)phosphonate hydrochloride

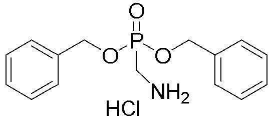

A mixture of diphenyl methyl ((triphenylmethylamino)methyl)phosphonate (2 g, 3.75 mmol) and HCl/dioxane (4 mol/L, 3.8 mL) in THF (40 mL) was stirred overnight at room temperature. The mixture was concentrated, to obtain a crude product (2.1 g, yield 90%). This crude product was directly used in the next step. MS (ESI): m/z = 292.2 [M+H]⁺.

### Step 5: 4-(N-(2-(((bis(benzyloxy)phosphoryl)methyl)carbamoyl)phenyl)aminosulfonyl)phenyl pivalate

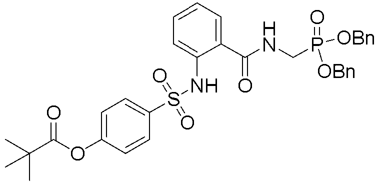

A mixture of 2-((4-(pivaloyloxy)phenyl)sulfonamido)benzoic acid (350 mg, 0.93 mmol), diphenyl methyl (aminomethyl)phosphonate hydrochloride (crude material 693 mg, 1.3 mmol), HOBt (125 mg, 0.93 mmol), DIPEA (479 mg, 3.7 mmol), and HBTU(457 mg, 1.2 mmol) in DCM (20 mL) was stirred at room temperature for 2 hrs. The mixture was concentrated and the residue was purified by column chromatography on silica gel (eluting with PE/EtOAc = 1/1, V/V), to obtain a product as a white solid (550 mg, yield 53%). MS (ESI): m/z = 651.3 [M+H]⁺.

### Step 6: ((2-((4-(pivaloyloxy)phenyl)sulfonamido)benzamido)methyl)phosphonic acid

The compound of Step 5 (520 mg, 0.8 mmol) and Pd/C (150 mg, 10%) were mixed with THF(20 mL) and added to a hydrogenation device. The system was evacuated and then refilled with hydrogen 4 times. The mixture was stirred at room temperature for 3 hrs. The reaction mixture was filtered. The filtrate was concentrated, directly purified by preparative HPLC (A: 0.1% TFA/water, B: CH₃CN; column: Xbridge BEH Peptide C18, 19 mm x 250 mm), and freezing-dried to obtain a compound 12 as a yellow solid (210 mg, 86%). MS (ESI): m/z = 471.1 [M+H]⁺.

¹H NMR (400 MHz, DMSO-d₆): δ 11.70 (s, 1 H), 9.02 (t, *J* = 5.8 Hz, 1 H), 8.70 (s, 2 H), 7.84 (d, *J* = 8.8 Hz, 2 H), 7.80 (d, *J* = 7.6 Hz, 1 H), 7.50-7.43 (m, 2 H), 7.30-7.28 (m, 2 H), 7.14-7.10 (m, 1 H), 3.58-3.53 (m, 2 H), 1.27 (s, 9 H).

### Example 13

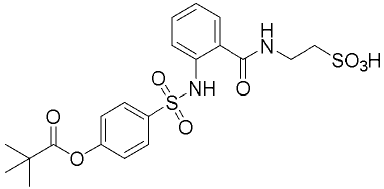

### Step 1: 2-(triphenylmethylthio)ethyl-1-amine

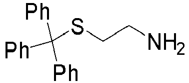

A mixture of 2-aminoethan-1-thiol hydrochloride (2 g, 17.6 mmol) and triphenylmethanol (4.58 g, 17.6 mmol) in TFA (15 mL) was stirred at room temperature for 1 hr. The solution was co-evaporated with toluene and the residue was dissolved in ethyl acetate (300 mL). The obtained mixture was adjusted to pH 8 with 1 M NaOH aqueous solution and washed with water (200 mL) and saturated NaCl solution (150 mL). The organic layer was dried over MgSO₄, filtered and concentrated, to obtain a crude product of 2-(triphenylmethylthio) ethyl-1-amine as a brown solid (5.7 g, yield 99%). This crude product was directly used in the next step.

MS (ESI): m/z = 342.0 [M+Na]⁺.

### Step 2: 2-amino-N-(2-(triphenylmethylthio)ethyl)benzamide

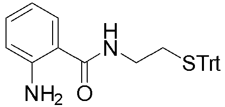

A mixture of 2-(triphenylmethylthio)ethyl-1-amine (2.94 g, 9.2 mmol), 2H-benzo[d][1,3]oxazin-2,4(1H)-dione (1.5 g, 9.2 mmol) and TEA (1.86 g, 18.4 mmol) in MeCN (30 mL) was heated to reflux for 4 hrs. The mixture was concentrated and purified by column chromatography on silica gel (eluting with EtOAc/PE = 1/3, V/V), to obtain the product 2-amino-N-(2-(triphenylmethylthio)ethyl)benzamide as a white solid (2 g, yield 50%). MS (ESI): m/z = 461.0 [M+Na]⁺.

### Step 3: 4-(N-(2-((2-(triphenylmethylthio)ethyl)carbamoyl)phenyl)aminosulfonyl)phenyl pivalate

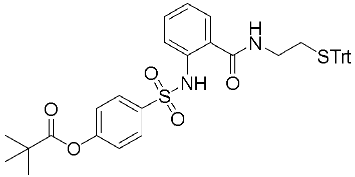

To a solution of 2-amino-N-(2-(triphenylmethylthio)ethyl)benzamide (500 mg, 1.14 mmol) and pyridine (2 mL) in DCM (15 mL), 4-(chlorosulfonyl)phenyl pivalate (379 mg, 1.37 mmol) was added and stirred overnight at room temperature. The mixture was concentrated and purified by preparative HPLC (A: 0.1% TFA/water, B: CH₃CN; column: Xbridge BEH Peptide C18, 19 mm x 250 mm), to obtain the product 4-(N-(2-((2-(triphenylmethylthio)ethyl)carbamoyl)phenyl)aminosulfonyl)phenyl pivalate as a white solid (700 mg, yield 91%). MS (ESI): m/z = 701.1 [M+Na]⁺.

### Step 4: 2-(2-((4-(pivaloyloxy)phenyl)sulfonamido)benzamido)ethan-1-sulfonic acid

A mixture of 4-(N-(2-((2-(triphenylmethylthio)ethyl)carbamoyl)phenyl)aminosulfonyl)phenyl pivalate (650 mg, 0.96 mmol), m-CPBA (1.65 g, 9.6 mmol) and NaOAc (197 mg, 2.4 mmol) in DCM (20 mL) was stirred at room temperature for 2 hrs. The mixture was concentrated, directly purified by preparative HPLC (A: 0.1% TFA/water, B: CH₃CN; column: Xbridge BEH Peptide C18, 19 mm x 250 mm), and freezing-dried to obtain a target compound 13 as a white solid (110 mg, yield 24%). MS (ESI): m/z = 485.1 [M+H]⁺.

¹H NMR (400 MHz, DMSO-d₆): δ 11.77 (s, 1 H), 8.83 (t, *J* = 5.0 Hz, 1 H), 7.81-7.78 (m, 2 H), 7.62 (d, *J* = 7.6 Hz, 1 H), 7.50-7.43 (m, 2 H), 7.30-7.27 (m, 2 H), 7.16-7.09 (m, 1 H), 3.50-3.46 (m, 2 H), 2.67 (t, *J* = 7.2 Hz, 2 H), 1.27 (s, 9 H).

### Example 14: Preparation of O-(methyl-d3)-N-(2-((4-(pivaloyloxy)phenyl)sulfonamido)benzoyl)-D-serine (25)

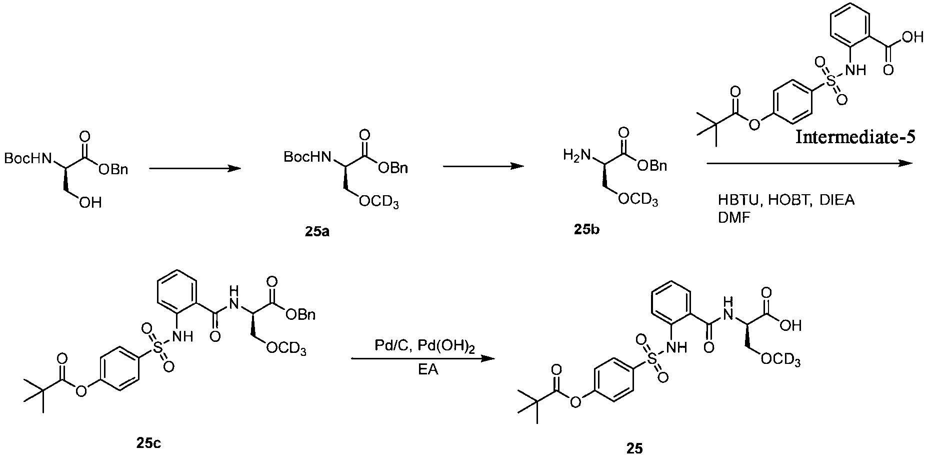

a) At room temperature and under nitrogen atmosphere, Ag₂O (588.5 mg, 2.5 mmol) and CD₃I (0.3 mL, 5.0 mmol) were sequentially added to a solution of benzyl(t-butoxycarbonyl)-D-serine (500.0 mg, 1.7 mmol) in MeCN (8 mL). The reaction solution was further stirred for 18 hrs in the dark. The reaction mixture was filtered. The filtrate was concentrated and purified by column chromatography on silica gel (eluting with 10 % EtOAc/PE, V/V), to obtain the product **25a** as a colorless liquid (400 mg, 76%). MS (ESI): m/z 212.7 [M-100]⁺.
b) At room temperature and under nitrogen atmosphere, HCl/Dioxane (4M, 3 mL) was added to **25a** (0.4 g, 1.3 mmol), and continuously stirred for 2 hrs. After concentration, the product **25b** as a white solid (300 mg, crude) was obtained. MS (ESI): m/z = 212.7 [M+H]⁺. The compound was used directly in the next reaction without purification.
c) At room temperature and under nitrogen atmosphere, HBTU (452 mg, 1.19 mmol, 1.3 eq.), HOBT (124 mg, 0.92 mmol, 1.0 eq.), and DIEA (1.18 g, 9.17 mmol, 10.0 eq.) were sequentially added to a solution of Intermediates **5** (346 mg, 0.92 mmol, 1.0 eq.) and **25b** (228 mg, 0.92 mmol, 1.0 eq.) in DCM (10 mL), and continuously stirred for 20 hrs. The reaction solution was concentrated and purified by column chromatography on silica gel (eluting with PE/EA 2:1, V/V) to obtain the product **25c** as a colorless oil (560 mg, 100%).
d) At room temperature, to a solution of **25c** (740 mg, 0.92 mmol, 1.0 eq.) in EtOAc (20 mL), 5% Pd/C (70 mg) and Pd(OH)₂ (70 mg) were added. At 40°C, hydrogenation was carried out and stirred for 16 hrs. After cooling to room temperature, the reaction mixture was filtered. The filtrate was concentrated and purified by reverse phase column chromatography to obtain the product **25** as a white solid (450 mg, 78%). (ESI): m/z = 481.75 [M+H]⁺. ¹H NMR (400 MHz, DMSO-d₆): δ 12.98 (s, 1H), 11.38 (s, 1H), 9.04 (d, *J* = 7.2 Hz, 1H), 7.83-7.78 (m, 3H), 7.51-7.48 (m, 2H), 7.30-7.27 (m, 2H), 7.19-7.15 (m, 1H), 4.62-4.58 (m, 1H), 3.77-3.66 (m, 2H), 1.27 (s, 9H).

### Biological activity test

Compound preparation: All compounds were dissolved in DMSO at a concentration of 10 mM, and stored at -20°C.

A 1x assay buffer was prepared. Serial 3-fold gradient dilutions of the positive compound (Sivelestat) and the test compounds were prepared in DMSO, and a total of 10 concentration points were obtained. 1 µL of the serially diluted compound and 65.67 µL of the assay buffer were added into a plate, to obtain 3X working solutions of the positive compound and the test compounds (the final concentration in DMSO was 1.5%). The plate was sealed with a sealing film and shaken on an oscillator for 15 min.

A 1x activation buffer was prepared. rhELA2 was diluted in the activation buffer containing DPPI and incubated at 37°C for 2 hrs to activate rhELA2. 5 µl of the 3x diluted working solutions of the compound was added to a 384-well plate. rhELA2 was diluted with the 1x assay buffer, to obtain a 3x working solution of rhELA2. 5 µl of the 3x working solution of rhELA2 was added to each well of the 384-well plate. The plate was sealed and incubated for 30 min at room temperature in the dark.

A 3x substrate solution was prepared in the 1x assay buffer. 5 µl of the 3x working solution of the substrate was added into the 384-well plate. The 384-well plate was centrifuged at 1000 rpm for 1 min, and incubated for 1 hr at room temperature in the dark. The fluorescence value FLU at 360 nm/450 nm was read on a plate reader.

The inhibition rate % was calculated. Inhibition rate =(1-(FLU of the compound-FLU of the positive well)/(FLU of the reference well-FLU of the positive well)). FLU of the reference well is that of a well with only the enzyme and no the inhibitor, and FLU of the positive well is that of a well with the enzyme and 3000 nm Sivelestat-2. Then the IC50 value was calculated by the formula Y=Bottom + (Top-Bottom)/(1+10^((LogIC50-X)*HillSlope)) using Graphpad 8.0 software.

| Compound No. | IC50 (nM) |
|---|---|
| 1 | 20.42 |
| 2 | 30.46 |
| 3 | 23.6 |
| 6 | 32.8 |
| 11 | 27.04 |
| 12 | 5.69 |
| 13 | 4.22 |
| 17 | 15.35 |
| 19 | 2.25 |
| 20 | 2.47 |
| 21 | 2.26 |
| 22 | 2.2 |
| 23 | 3.69 |
| 24 | 1.5 |
| 25 | 2.54 |
| 26 | 8.49 |
| 27 | 130.5 |

The compounds of the present invention has an IC50 of less than 100 nM; particularly an IC50 of less than 50 nM; and more preferred compounds have an IC50 of less than 10 nM. The compounds of the present invention show excellent inhibitory activity.

### Animal test 1

Test animals: ICR mice, SPF grade, male, weight at the time of administration: 25-28 g.

Animal grouping: 4 groups, each group having 10 animals. The four groups were respectively a blank control group, a model group, a test group (Compound 20) and a positive control group (Sivelestat sodium).

Establishment of mouse model of LPS-induced acute lung injury: The mice were anesthetized by intramuscular injection of Zoletil^{®}50 (0.05 mL/mouse). Then the mice were fixed in a supine position, the cervical trachea was isolated, and lipopolysaccharide (LPS) (1 mg/mL, 35 µL/mouse) was sprayed by a high-pressure atomizing nozzle. The neck skin was sutured, and disinfected with iodophor.

Dosage of administration: The test group and the positive control group were given Compound 20 or Sivelestat sodium once by intravenous injection 0.5 h, 4 h and 12 h after model establishment with LPS. The dosage at each administration was 25 mg/kg, and the total daily dosage was 75 mg/kg.

24 h after LPS was sprayed into the trachea of mice, the mice were sacrificed, and the trachea was isolated and intubated. The left lung lobe was ligated. The lung lavage was carried out with 0.5 mL normal saline (containing 1% serum and 2 U/mL heparin), and then the process was repeated two times. The lavage fluids of the 3 times were mixed and stood on ice for later use. The ligated left lung lobe was removed from the animal Nos. 1-8 in the blank control group, the model group, the test group and the positive control group respectively, weighed, added with 0.9% NaCl at a ratio of 1:6, homogenized at 4°C and centrifuged at 10000 g for 10 min. The supernatant was collected and the contents of inflammatory factors IL-1β and TNF-α were determined. The results of each group are shown in the table below.

| Grouping and administration | Content of inflammatory factor (pg/mL, Mean±SD) | |
|---|---|---|
| | IL-1β | TNF-α |
| Blank control group | 488±217 | 317±102 |
| Model group | 1290±341 ^{###} | 762±248 ^{##} |
| Test group (Compound 20, 75mg/kg) | 784±159 ^{**} | 442±118 ^{*} |
| Positive control group (Sivelestat sodium, 75 mg/kg) | 1048±384 | 552±77 ^{*} |

Note: Compared with the blank control group: ^{##} *P<0.01,^{###} P<0.001*; compared with the model group: * *P<0.05, ^{**} P<0.01.*

In the mouse model of LPS-induced acute lung injury, the inhibitory effects of Compound 20 on inflammatory factors IL-1β and TNF-α are significantly better than those of Sivelestat sodium.

### Animal test 2

Animal grouping: 4 groups, each group having 8 animals. The four groups were respectively a blank control group, a model group, a test group (Compound 20) and a positive control group (Sivelestat sodium).

Establishment of rat model of hydrochloric acid-induced acute lung injury: 0.1 N HCL was sprayed into the trachea of rats at a dosage of 0.1 mL/rat.

Dosage of administration: The test group was given Compound 20, and the positive control group was given Sivelestat sodium. Both groups were administered twice by tail vein injection, immediately after the model establishment and again 2.5 hrs after the model establishment. The total dosage of Compound 20 in the test group was 120 mg/kg and the total dose of Sivelestat sodium in the positive control group was 30 mg/kg.

The rats were sacrificed 5 h after the model establishment. Alveolar lavage was performed. The inflammatory cells, protein exudation, pulmonary hemorrhage and NE enzyme activity in the lavage fluid were measured. The results of each group are shown in the table below.

| Grouping and administration | Pulmonary hemorrhage (IOD, absorbance at 412 nm) | Protein content in alveolar lavage fluid (mg/mL) | White blood cell count (WBC, 1×10⁶ cell/mL) |
|---|---|---|---|
| Blank control group | 0.09±0.02 | 0.09±0.04 | 0.09±0.02 |
| Model group | 0.39±0.18 ^{###} | 0.49±0.09 ^{###} | 0.49±0.18 ^{##} |
| Test group (Compound 20, 120 mg/kg) | 0.20±0.03 ^{**} | 0.30±0.05 ^{***} | 0.28±0.10 ^{**} |
| Positive control group (Sivelestat sodium, 30 mg/kg) | 0.25±0.10 ^{*} | 0.35±0.07 ^{**} | 0.42±0.11 |

Note: Compared with the blank control group: ^{##} *P<0.01,^{###} P<0.001*; compared with the model group: *^{*} P<0.05, ^{**} P<0.01, ^{***} P<0.001.*

In the rat model of hydrochloric acid-induced acute lung injury, Compound 20 improves pulmonary hemorrhage and reduces the protein exudation and white blood cell count in the alveolar lavage fluid more obviously compared with Sivelestat sodium.

## Claims

1. A compound of Formula I or a pharmaceutically acceptable salt thereof, 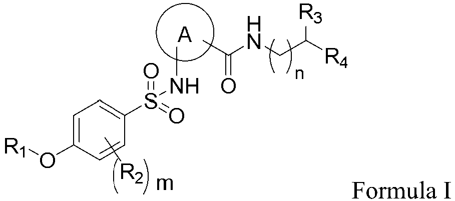 wherein
R₁ is selected from R₆-C(=O)-, or R₁ is attached to a benzene ring to form a five-membered or six-membered ring, in which the five-membered or six-membered ring is optionally substituted with R₆;
R₆ is selected from t-butyl, C₃-C₆ cycloalkyl, and C₃-C₆ heterocycloalkyl;
ring A is selected from 5 to 10-membered cycloalkyl, heterocycloalkyl, heteroaryl, and aryl, ring A is optionally substituted with one or more R₅;
R₂ and R₅ are each independently selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl;
R₃ is selected from hydrogen, C₁-C₆ alkyl, C₁-C₆ alkoxy, C₃-C₆ cycloalkyl, and C₁-C₆ haloalkyl;
R₄ is optionally selected from -COOH, -SO₃H, and -PO₃H₂; and
m and n are independently 0, 1, 2, or 3;
where the alkyl, alkoxy, cycloalkyl, heterocycloalkyl, and heteroaryl are unsubstituted or optionally substituted with one or more deuterium, C₁-C₃ alkyl, C₁-C₃ alkoxy, C₁-C₃ deuterated alkoxy, halogen, cyano, trifluoromethyl, nitro, amino, -CONH₂, and -COOH.

2. The compound of Formula I or the pharmaceutically acceptable salt thereof according to claim 1, wherein Formula I has a structure of Formula I-a: 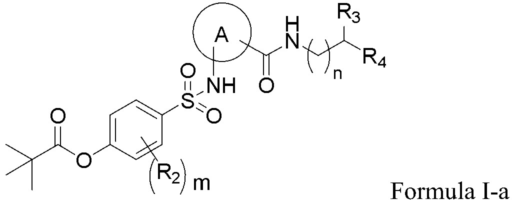 in which
ring A is phenyl, pyrazolyl, imidazolyl, pyrazinyl, or cyclohexyl, and is optionally substituted with one or more R₅;
R₄ is selected from -COOH, -SO₃H, and -PO₃H₂; and
R₂, R₃, R₅, m, and n are as defined in claim 1.

3. The compound of Formula I or the pharmaceutically acceptable salt thereof according to claim 1, wherein Formula I has a structure of Formula I-b: 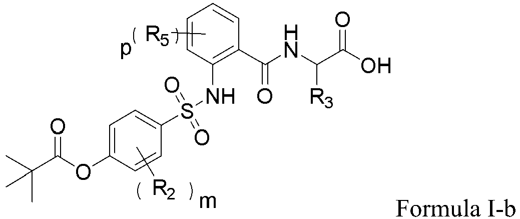
in which R₂, R₃, R₅ and m are as defined in claim 1, and p is 0, 1 or 2;
provided that in Formula I-b, R₂, R₃ and R₅ are not all hydrogen.

4. The compound of Formula I or the pharmaceutically acceptable salt thereof according to claim 1, wherein Formula I has a structure of Formula I-c: 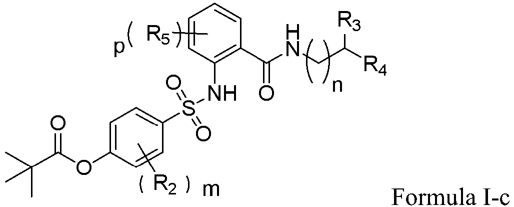
in which R₄ is optionally selected from -SO₃H and -PO₃H₂; and
R₂ and R₅ are each independently selected from hydrogen, halogen, cyano, trifluoromethyl, nitro, amino, C₁-C₆ alkyl, C₁-C₆ alkoxy, C₃-C₆ cycloalkyl, C₃-C₆ heterocycloalkyl, and C₃-C₆ heteroaryl;
R₃ is selected from hydrogen, C₁-C₆ alkyl, C₁-C₆ alkoxy, C₃-C₆ cycloalkyl, and C₁-C₆ haloalkyl; and
n, m, and p are each independently 0, 1 or 2.

5. The compound of Formula I or the pharmaceutically acceptable salt thereof according to claim 1, wherein R₂ is optionally selected from hydrogen and fluorine.

6. The compound of Formula I or the pharmaceutically acceptable salt thereof according to claim 1, wherein R₃ is optionally selected from hydrogen, methyl, fluoromethyl, methoxymethyl, and deuterated methoxymethyl.

7. The compound of Formula I or the pharmaceutically acceptable salt thereof according to claim 1, wherein R₅ is optionally selected from hydrogen, fluorine, methyl, cyano, methoxy, imidazolyl, pyrazolyl, oxazolyl, and isoxazolyl, wherein imidazolyl, pyrazolyl, oxazolyl, and isoxazolyl are unsubstituted or substituted with methyl.

8. The compound of Formula I or the pharmaceutically acceptable salt thereof according to claim 1, which is selected from the following compounds:

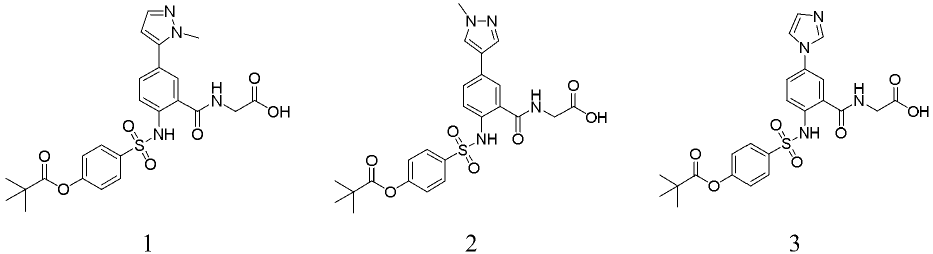

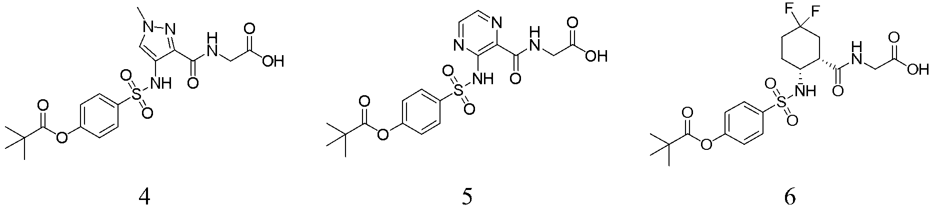

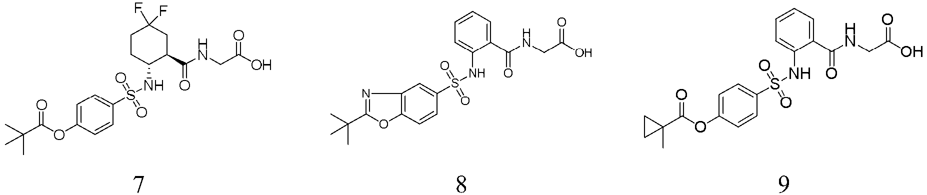

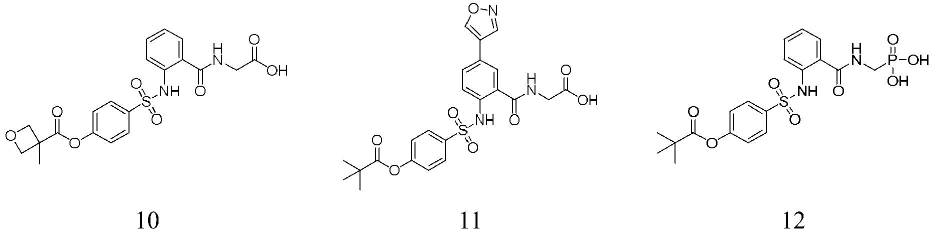

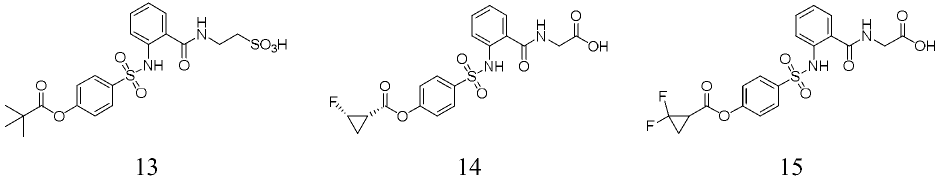

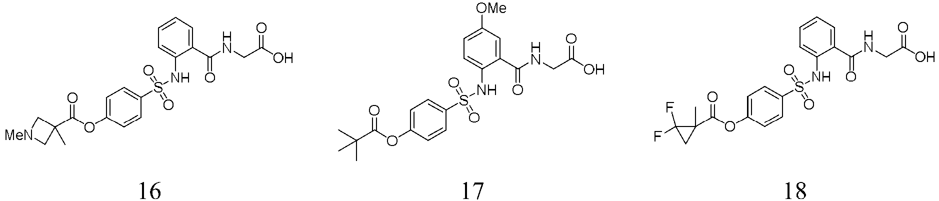

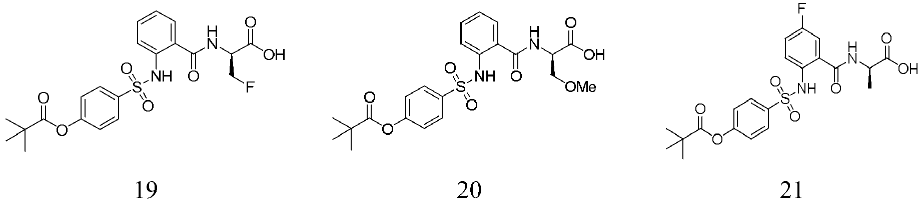

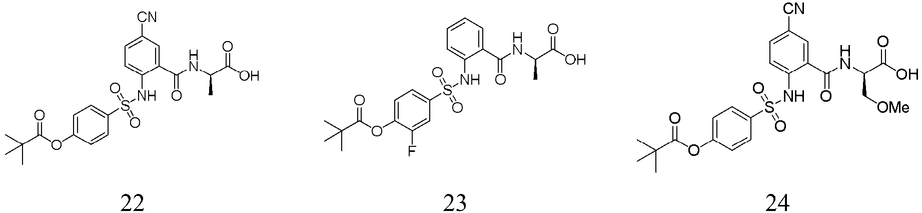

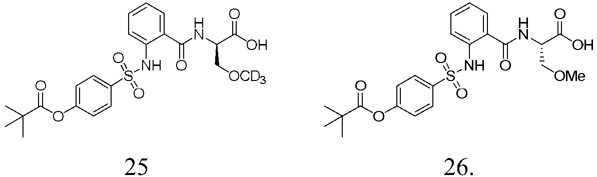

9. A pharmaceutical composition, comprising a compound of Formula I or a pharmaceutically acceptable salt thereof and a pharmaceutically acceptable adjuvant.

10. Use of the compound or the pharmaceutically acceptable salt thereof according to claim 1 or the pharmaceutical composition according to claim 9 in the preparation of a drug for treating or preventing diseases caused by abnormal increase of neutrophil elastase.

11. The use according to claim 10, wherein the diseases comprise autoimmune inflammation, infectious inflammation, traumatic inflammation, allergic inflammation, systemic inflammation, respiratory inflammation, lung inflammation and pain.
